(19) **Europäisches Patentamt European Patent Office Office européen des brevets**

(11) **EP 1 358 000 B1**

(12) **EUROPEAN PATENT SPECIFICATION**

(45) Date of publication and mention of the grant of the patent:
**25.04.2007 Bulletin 2007/17**

(51) Int Cl.:
**B01D 57/02** (2006.01)  **G01N 27/447** (2006.01)
**B01D 61/42** (2006.01)  **C25B 7/00** (2006.01)

(21) Application number: **01271253.5**

(22) Date of filing: **21.12.2001**

(86) International application number:
**PCT/AU2001/001669**

(87) International publication number:
**WO 2002/049744 (27.06.2002 Gazette 2002/26)**

(54) **APPARATUS AND METHOD FOR SEPARATION OF MOLECULES AND MOVEMENT OF FLUIDS BY ELECTROPHORESIS**

VORRICHTUNG UND VERFAHREN ZUR TRENNUNG VON MOLEKÜLEN UND BEWEGUNG VON FLUIDEN MITTELS ELEKTROPHORESE

APPAREIL ET PROCEDE POUR LA SEPARATION DE MOLECULES ET LE DEPLACEMENT DE FLUIDES PAR ÉLECTROPHORÈSE

(84) Designated Contracting States:
**AT BE CH CY DE DK ES FI FR GB GR IE IT LI LU MC NL PT SE TR**

(30) Priority: **21.12.2000 AU PR222400**

(43) Date of publication of application:
**05.11.2003 Bulletin 2003/45**

(73) Proprietor: **Life Therapeutics Limited Frenchs Forest, NSW 2086 (AU)**

(72) Inventors:
• **ROETH, Philip, John**
  **Castle Hill, NSW 2154 (AU)**
• **BOTTO, Steven, Anthony**
  **Rydalmere, NSW 2116 (AU)**
• **CURLEY, Benjamin, John**
  **Sutherland, NSW 2232 (AU)**
• **NAIR, Chenicheri, Hariharan**
  **Old Greenwich, CT 06870 (US)**

(74) Representative: **Elend, Almut Susanne et al Venner Shipley LLP Byron House Cambridge Business Park Cowley Road Cambridge CB4 0WZ (GB)**

(56) References cited:
**EP-A- 0 369 945**       **WO-A-00/13776**
**US-A- 3 870 617**      **US-A- 4 441 978**
**US-A- 4 608 147**

**Description**

Technical Field

The present invention relates to method and apparatus for the separation of compounds, particularly macromolecules, where bulk movement of liquids can be managed.

Background Art

Membrane-based electrophoresis is a new technology originally developed for the separation of macromolecules such as proteins, nucleotides and complex sugars. The process provides a high purity, scalable separation that is faster, cheaper and higher yielding than current methods of macromolecule separation and offers the potential to concurrently purify and detoxify macromolecule solutions.

In one form, the technology is bundled into a cartridge comprising a number of membranes housed in a system which allows separation of compounds by charge and/or molecular mass. The system can also concentrate and desalt/dialyse at the same time. The multimodal nature of the system allows this technology to be used in a number of other areas especially in the production of biological components for medical use. The structure of the membranes may be configured so that biological contaminants can also be removed at the point of separation - a task which is not currently available in the biotechnology industry and which adds to the cost of production through time delays and due to the complexity of the task.

For example, US 3,870,617 discloses an apparatus for continuous forced flow electrophoresis and its use, the apparatus comprising conventional microporous, dialysis and ion-selective membranes suitable for the fractionation of blood under the influence of an electric filed and a hydrostatic pressure difference. US 4,608,147 meanwhile discloses an apparatus for operation in an electrophoresis chamber for the electro-elution of biological molecules from sections of electrophoresis gels, the apparatus achieving semi-purification of the biological molecules by means of a trap between two membranes permeable to all low molecular weight ions and molecules. US 4,441,978 discloses a two-stage apparatus in which a liquid protein mixture is first demineralised by electrodialysis using conventional ion-selective membranes and then subjected to isoelectric focusing to effect the separation of proteins based on differences in their isoelectric points.

The transfer of liquid from on area to another, through a porous membrane, under electrophoretic conditions, is called electro-endo-osmosis (EEO). Electro-endo-osmosis is a natural occurrence with membrane-based electrophoresis technology and its management can result in the increase of product recovery, decrease in run times and concentrate samples. These improvements can be achieved by maintaining the concentration of the target molecule, in a specific steam, by managing the extent of bulk liquid transfer. One method to manage electro-endo-osmosis was via electro-osmosis, where an external power source alters the rate of a system undergoing osmosis or endo-osmosis. The impact of large volume increase is potentially more serious in the scale-up use of the system. Control of electro-endo-osmosis would contribute significantly to cost reduction and efficiencies in plant maintenance. The ability to concentrate samples without adversely affecting activity, functionality, quantity, for example would be useful.

The present inventors have now developed a modification of the membrane-based electrophoresis technology to assist in the management of bulk liquid transfer/endo-osmosis.

Disclosure of Invention

In a first aspect, the present invention provides an electrophoresis apparatus according to claim 1 and comprising:

(a) a first electrode in a first electrode zone;
(b) a second electrode in a second electrode zone, the first electrode disposed relative to the second electrode so as to be adapted to generate an electric field in an electric field area therebetween upon application of an electric potential between the first and second electrodes;
(c) a first membrane disposed in the electric field area;
(d) a second membrane disposed between the first electrode zone and the first membrane so as to define a first interstitial volume therebetween; wherein the first interstitial volume is separated from the first and second electrode zones by the first and second membranes;
(e) means adapted to communicate liquid to the first electrode zone and the second electrode zone; and
(f) means adapted to communicate a sample constituent to the first interstitial volume

characterised in that at least one membrane is a barrier in the form of an inducible electro-endo-osmotic membrane capable of controlling substantial bulk movement of liquid under the influence of an electric field, wherein the at least one inducible electro-endo-osmotic membrane is either:

1) made from cellulose triacetate with a molecular mass cut-off from 5 to 20 kDa, or

2) made from polyvinyl alcohol;

In use, a sample is placed in the first interstitial volume (also called stream 1), buffer or solvent is provided to the electrode zones, an electric potential is applied to the electric field area causing movement of water out of the sample to the an adjacent electrode zone. The sample is thereby concentrated by driving liquid out of the sample. The membrane forming the barrier substantially prevents bulk movement of liquid into the sample and the electrophoresis process causes water and salts to move out of the sample.

It is also feasible to place sample in one (or both) of the electrode zones and cause movement of one or more a compounds from the sample into the adjacent interstitial volume during the application of the voltage potential.

The apparatus may further comprise:

means adapted to apply a selected electric potential across at least the electric field area. In this form, a power supply is provided or integrated with the apparatus. Typically, the apparatus is connected to an external power supply by any suitable electrical connector means.

In a first embodiment the present invention provides an electrophoresis apparatus comprising:

(a) a first electrode in a first electrode zone;
(b) a second electrode in a second electrode zone, the first electrode disposed relative to the second electrode so as to be adapted to generate an electric field in an electric field area therebetween upon application of an electric potential between the first and second electrodes;
(c) a first membrane disposed in the electric field area;
(d) a second membrane disposed between the first electrode zone and the first membrane so as to define a first interstitial volume therebetween;
(e) a third membrane disposed between a second electrode zone and the first membrane so as to define a second interstitial volume therebetween; wherein the first interstitial volume is separated from the first electrode zone by the second membrane and the second interstitial volume is separated from the second electrode zone by the third membrane; and wherein at least one membrane being a barrier capable of controlling substantial bulk movement of liquid under the influence of an electric field;
(f) means adapted to communicate liquid to the first electrode zone, the second electrode zone and the second interstitial volume;
(g) means adapted to communicate a sample constituent to the first interstitial volume wherein, upon application of the electric potential, one or more components is caused to move from the sample constituent through at least one membrane to an adjacent electrode zone or an interstitial volume and at least one membrane controls substantial bulk movement of liquid into or out of the sample..
In one preferred form, the apparatus further comprises:-
(h) means adapted for removing heat generated in the apparatus.

Preferably, sample and liquid are passed through heat exchangers to remove heat produced by the apparatus during electrophoresis.

The apparatus may further comprise:

means adapted to apply a selected electric potential across at least the electric field area. In this form, a power supply is provided or integrated with the apparatus. Typically, the apparatus is connected to an external power supply by any suitable electrical connector means.

In a second embodiment, the present invention provides an electrophoresis system comprising:

(a) a first electrode in a first electrode zone;
(b) a second electrode in a second electrode zone, the first electrode disposed relative to the second electrode so as to be adapted to generate an electric field in a first electric field area therebetween upon application of a first electric potential between the first and second electrodes;
(c) a first membrane disposed in the electric field area;
(d) a second membrane disposed between the first electrode zone and the first membrane so as to define a first interstitial volume therebetween;

(e) a third membrane disposed between the second electrode zone and the first membrane so as to define a second interstitial volume therebetween;

(f) means adapted to communicate liquid to the first electrode zone, the second electrode zone and the second interstitial volume;

(g) means adapted to communicate a sample constituent to one of the first or second interstitial volumes; wherein upon application of the first-electric potential, one or more components is caused to move from the sample constituent through at least one membrane to the adjacent other of the first or second interstitial volumes;

(h) a third electrode in a third electrode zone;

(i) a fourth electrode in a fourth electrode zone, the third electrode disposed relative to the fourth electrode so as to be adapted to generate an electric field in a second electric field area therebetween upon application of a second electric potential between the third and fourth electrodes;

(j) a fourth membrane disposed in the second electric field area;

(k) a fifth membrane disposed between the third electrode zone and the fourth membrane so as to define a third interstitial volume therebetween; wherein the fourth interstitial volume is separated from the third and fourth electrode zones by the fourth and fifth membranes, and wherein at least one of the fourth or fifth membranes being a barrier capable of controlling substantial bulk movement of liquid under the influence of an electric field;

(l) means adapted to communicate liquid to the third electrode zone and the fourth electrode zone; and

(m) means adapted to communicate a sample constituent from the first or second interstitial volume to the third interstitial volume; wherein, upon application of the second electric potential, liquid is caused to move from the sample constituent in the third interstitial volume through at least the forth or fifth membranes to an adjacent electrode zone; wherein the barrier membrane prevents substantial bulk movement of liquid into the sample.

In one preferred form, the apparatus further comprises:-

(n) means adapted for removing heat generated in the apparatus.

Preferably, sample and liquid are passed through heat exchangers to remove heat produced by the apparatus during electrophoresis.

The apparatus may further comprise:

means adapted to apply a first selected electric potential across the first electric field area; and

means adapted to apply a second selected electric potential across the second electric field area. In this form, one or more power supplies is provided or integrated with the apparatus. Typically, the apparatus is connected to a external power supply by any suitable electrical connector means such that two separate electric potentials can be applied.

In another preferred form, some of the membranes are electrophoresis separation membranes and other membranes are restriction membranes having defined pore sizes. Preferably, the restriction membranes are positioned between the electrode zones and an adjacent interstitial volume. At least one of the restriction membranes is the inducible electro-endo-osmotic membrane which controls the substantial bulk movement of liquid under the influence of an electric field.

The electrophoresis separation membranes are preferably made from polyacrylamide and have a molecular mass cut-off of at least about 3 kDa. The molecular mass cut-off of the membrane will depend on the sample being processed, the other molecules in the sample mixture, and the type of separation carried out.

At least one restriction membrane is also preferably formed from polyacrylamide. The molecular mass cut-off of the restriction membrane will depend on the sample being processed, the other molecules in the sample mixture, and the type of separation carried out.

The inducible electro-endo-osmotic membrane is preferably a cellulose tri-acetate (CTA) membrane. It will be appreciated that the inducible electro-endo-osmotic membrane can be formed from any other suitable membrane material such as poly(vinyl alcohol) cross-linked with glutaraldehyde (PVAl+glut).

The present inventors have found that a CTA membrane having a nominal molecular mass cut-off of 5, 10 or 20 kDa are particularly suitable for use in the apparatus according to the present invention.

The cathode zone and the anode zone are supplied with suitable electrolyte or buffer solutions by any suitable pumping means. A sample to be processed is supplied directly to the first interstitial volume or second interstitial volume (if present) by any suitable pumping means.

Preferably, the zones and the interstitial volume(s) are configured to allow flow of the respective liquid/buffer and sample solutions forming streams. In this form, large volumes can be processed quickly and efficiently. The solutions are typically moved or recirculated through the zones and interstitial volume(s) from respective reservoirs by suitable pumping means. In a preferred embodiment, peristaltic pumps are used as the pumping means for moving the sample, buffers or liquids.

Electrode buffers or electrolytes and sample buffers can be any suitable buffer or electrolyte. Examples include but not limited to Tris/Borate, Hepes/Imidazole, GABA/Acetic acid and Hepes/Histidine buffers.

The present invention is suitable for the separation or treatment of any compound capable of having a charge or a defined molecular mass. Examples include but not limited to biological compounds such as peptides, proteins, nucleic acids, and the like.

In one embodiment, electrode buffer, other buffers and sample solutions are cooled by any suitable means to ensure no inactivation of the micromolecules, compounds, macromolecules or other sample components occurs during the electrophoresis process and to maintain a desired temperature of the apparatus while in use.

Preferably, in order to collect and/or concentrate sample components, liquid in at least one of the volumes or streams containing any separated components or molecules is collected and replaced with suitable solvent to ensure that electrophoresis can continue in an efficient manner.

In use, a sample is placed in the first interstitial volume (also called stream 1), buffer or solvent is provided to the electrode zones arid the second interstitial volume (stream 2), an electric potential is applied to the electric field area causing at least one constituent in the sample to move to buffer/solvent in the adjacent electrode zone or to the adjacent second interstitial volume. The membrane forming the barrier substantially prevents movement of liquid into the sample.

It will be appreciated that the order of interstitial volumes can be reversed where a sample is placed in the second interstitial volume (stream 2), buffer or solvent is provided to the electrode zones and the first interstitial volume, an electric potential is applied to the electric field area causing at least one constituent in the sample to move to the adjacent electrode zone or to the adjacent first interstitial volume (stream 1).

It is also feasible to place a sample in one (or both) of the electrode zones and cause movement of one or more compounds into one or more of the interstitial volumes during the application of the voltage potential.

The first membrane is preferably an electrophoresis separation membrane comprised of polyacrylamide (PA) and having a defined molecular mass cut-off. Preferably, the electrophoresis separation membrane has a molecular mass cut-off from about 1 kDa to about 2000 kDa. The selection of the molecular mass cut-off of the separation membrane will depend on the sample being processed and the other molecules in the mixture. It will be appreciated, however, that other membrane chemistries or constituents can be used for the present invention.

At least one of the second and third membranes is a restriction membrane preferably formed from polyacrylamide and usually having a molecular mass cut-off less than the separation membrane, preferably from about 1 kDa to about 1000 kDa. The selection of the molecular mass cut-off of the restriction membrane will depend on the sample being processed and the size of the macromolecules to be removed. The restriction membrane can have the same molecular mass cut-off or different cut-off from that of the membrane forming the barrier.

In one preferred form, the membranes forming the first and second interstitial volumes are provided as a cartridge or cassette positioned between the electrode zones of the apparatus. The configuration of the cartridge is preferably a housing with the first membrane positioned between the second and third membranes thus forming the required interstitial volumes.

Preferably, the cartridge or cassette is removable from an electrophoresis apparatus adapted to contain or receive the cartridge.

The cartridge may also include one or more of the electrodes.

The distance between the electrodes has an effect on the separation or movement of sample constituents through the membranes. The shorter the distance between the electrodes, the faster the electrophoretic movement of constituents. A distance of about 6 mm has been found to be suitable for a laboratory scale apparatus. For scale up versions, the distance will depend on the number and type of separation membranes, the size and volume of the chambers for samples, buffers and separated products. Preferred distances would be in the order of about 6 mm to about 10 cm. The distance will also relate to the voltage applied to the apparatus.

The effect of the electric field is based on the equation:

$$e = V/d$$

(e = electric field, V = voltage, d = distance)

Therefore, the smaller the distance between the electrodes, the faster the separation. Preferably, the distance between the electrodes should decrease in order to increase electric field strength, thereby further improving transfer rates through the membranes.

Flow rate of sample/buffer/liquid has an influence on the separation of constituents. Rates of millilitres per minute up to litres per minute are used depending on the configuration of the apparatus and the nature and volume of the sample to be separated. Currently in a laboratory scale instrument, the preferred flow rate is about $20 \pm 5$ mL/min.

However, flow rates from about 0 mL/min to about 50,000 mL/min are used across the various separation regimes. The maximum flow rate is even higher, depending on the pumping means and size of the apparatus. The selection of the flow rate is dependent on the product to be transferred, efficiency of transfer, pre- and post- positioning with other applications.

Selection or application of the voltage and/or current applied varies depending on the separation. Typically up to several thousand volts are used but choice and variation of voltage will depend on the configuration of the apparatus, buffers and the sample to be separated. In a laboratory scale instrument, the preferred voltage is about 250 V. However, depending on transfer, efficiency, scale-up and particular method from about 0 V to about 5000 V are used. Higher voltages are also considered, depending on the apparatus and sample to be treated.

Optionally, the electric potential may be periodically stopped and/or reversed to cause movement of a constituent having entered a membrane to move back into the volume or stream from which it came, while substantially not causing any constituents that have passed completely through a membrane to pass back through the membrane.

Reversal of the electric potential is an option but another alternative is a resting period. Resting (a period without an electric potential being applied) is an optional step that can replace or be included before or after an optional electrical potential reversal. This resting technique can often be practised for specific separation applications as an alternative or adjunct to reversing the potential.

For convenience, the first interstitial volume or stream is called stream 1 and the second interstitial volume or stream is called stream 2. Typically, sample was placed in stream 1 and constituents caused to move through the separation membrane into stream 2.

In one particularly preferred embodiment, the electrodes are made of titanium mesh coated with platinum. It will be appreciated, however, that other materials and configurations can be used.

In use, buffer or other suitable solvent is circulated through the electrode zones and liquid sample is applied to at least one of the first and second interstitial volumes. When an electric potential or field is applied to the apparatus via the electrodes, some components in the sample will be caused to move through the membrane into the adjacent interstitial volume or an electrode zone. The inducible electro-endo-osmotic membrane prevents or controls substantial bulk liquid movement between the interstitial volume and electrode zone thereby preventing undesirable dilution of the sample or separated compound during the separation process.

The membranes may be formed as a multilayer or sandwich arrangement. The thickness of the membranes can have an effect on the separation or movement of compounds. It has been found that the thinner the membrane, the faster and more efficient movement occurs.

In a second aspect, the present invention provides a method for concentrating a sample according to claim 11. In one embodiment the method comprises:

(a) providing an electrophoresis apparatus according to the first aspect of the present invention;
(b) adding buffer or electrolyte to the first and second electrode zones;
(c) adding the sample to the first interstitial volume;
(d) applying an electric potential between the electrodes causing liquid in the sample to move through a membrane into an adjacent electrode volume; and optionally
(f) obtaining the concentrated sample;

wherein the barrier membrane allows bulk movement of liquid out of the sample.

In a further embodiment, the present invention provides a method for moving at least one component from a sample while controlling the bulk movement of liquid, the method comprising:-

(a) providing an electrophoresis apparatus according to the first embodiment of the first aspect of the present invention;
(b) adding buffer or electrolyte to the first and second electrode zones;
(c) adding the sample to the first interstitial volume;
(d) adding buffer or liquid to the second interstitial volume;
(e) applying an electric potential between the electrodes causing at least one compound from the sample to move through a membrane into an adjacent electrode zone or interstitial volume; and optionally
(f) collecting the compound moved from the sample;

wherein the barrier membrane controls substantial bulk movement of liquid into or out of one or more of the interstitial volumes.

In still a further embodiment, the present invention provides a method for moving at least one component from a sample while controlling the bulk movement of liquid, the method comprising:-

(a) providing an electrophoresis system according to the second embodiment of the first aspect of the present invention;

(b) adding buffer or electrolyte to the first, second, third and fourth electrode zones;

(c) adding the sample to the first interstitial volume;

(d) adding buffer or liquid to the second interstitial volume;

(e) applying a first electric potential between the first and second electrodes causing at least one compound from the sample to move through a membrane into the second interstitial volume;

(f) passing sample containing the compound from the second interstitial volume to the third interstitial volume;

(g) applying a second electric potential between the third and fourth electrodes causing liquid in the third interstitial volume to move through a membrane into an adjacent electrode zone thereby concentrating the compound in the sample; and optionally

(h) collecting the concentrated sample; wherein the barrier membrane controls substantial bulk movement of liquid into or out of the third interstitial volume.

The barrier membrane can prevent substantial bulk movement of liquid into one or more of the interstitial volumes or can cause the movement of liquid out of an interstitial volume.

In one preferred from, two apparatus are connected in a manner so as to further control or prevent bulk movement of liquid into one or more of the interstitial volumes. The first apparatus is configured according to the first embodiment of the first aspect of the present invention with or without the barrier membrane and functions to separate one or more compounds of choice. The second apparatus can be configured according to the first aspect of the present invention and allows concentration of the separated compound. Preferably, the electric potential applied to each apparatus is under separate control. In one preferred form, the second interstitial volume of the first apparatus is in fluid communication with the first interstitial volume of the second apparatus. In use, after the compound to be separated is caused to move to the second interstitial volume of the first apparatus, it is then transferred to the first interstitial volume of the second apparatus which causes unwanted liquid to move out of the separated compound. This dual apparatus system functions as a further sample concentrator without the substantial loss of liquid or sample.

In another preferred form, two apparatus are connected in a manner so as to further control or prevent bulk movement of liquid into one or more of the interstitial volumes. The first apparatus is configured according to the first embodiment of the first aspect of the present invention with or without the barrier membrane and functions to separate one or more compounds of choice. The second apparatus is configured according to the first aspect of the present invention and allows concentration of the sample during the electrophoresis. Preferably, the electric potential applied to each apparatus is under separate control. In this preferred form, the first interstitial volume of the first apparatus containing the sample is in fluid communication with the first interstitial volume of the second apparatus. In use, the sample is circulated between the two apparatus to remove or reduce liquid build-up caused during electrophoresis in the first apparatus. The compound to be separated is caused to move to the second interstitial volume of the first apparatus for collection. This dual apparatus system functions as a further sample concentrator without the substantial loss of liquid or sample. The present invention relates to use of the apparatus according to the first aspect of the present invention in the separation or electrophoresis processing of one or more compounds from a sample. The present invention relates to use of the apparatus according to the second embodiment of the first aspect of the present invention in the separation or electrophoresis processing of one or more compounds from a sample.

Gradiflow™ is a trade mark owned by Gradipore Limited, Australia.

In order that the present invention may be more clearly understood, preferred forms will be described with reference to the following drawings and examples.

Brief Description of the Drawings

Figure 1 is a schematic diagram of a first embodiment of the present invention. Figure 1A shows an arrangement of electrodes, electrode zones and interstitial volume. Figure 1B shows positioning of two membranes in relation to electrodes.

Figure 2 is a schematic diagram of a second embodiment of the present invention. Figure 2A shows an arrangement of electrodes, electrode zones and first and second interstitial volumes. Figure 2B shows positioning of three membranes in relation to electrodes.

Figure 3 is a schematic diagram of a third embodiment of the present invention. Figure 3A shows an arrangement of electrodes, electrode zones and first, second and third interstitial volumes. Figure 3B shows positioning of five membranes in relation to two sets of electrodes.

Figure 4 shows PAGE of CTA calibration experiments.

Figure 5 shows endo-osmosis rates with CTA membranes.

Figure 6 shows comparison of CTA orientation and the endo-osmotic rate.

Figure 7 shows rate of volume removal due to electro-endo-osmosis using CTA membranes.

Figure 8 shows bovine serum albumin (BSA) recovery with voltage change.

Figure 9 shows rate of volume removal due to electro-endo-osmosis with PVAI membranes.

Figure 10 shows rate of volume removal due to electro-endo-osmosis

Figure 11 shows plumbing of two apparatus incorporating a stream 1 concentrator machine for the management of endo-osmosis.

Figure 12 shows comparison of endo-osmotic rate and percentage fibrinogen recovery.

Figure 13 shows PAGE analysis of simultaneous separation and concentration of bovine Prion Protein (PrP) of bovine brain homogenate using membrane-based electrophoresis technology and PVAI membrane. Part A: SDS-PAGE of the samples from the electrophoresis run; Part B: Western blot of the samples from the electrophoresis run using anti-PrP R029 (Prionics, Switzerland). $S1_0$ Stream 1 at time 0 minutes; $S1_{180}$ Stream 1 at time 180 minutes; $S2_0$ Stream 2 at time 0 minutes; $S2_{180}$ Stream 2 at time 180 minutes.

Figure 14 shows a first configuration of the present invention which allows for simultaneous concentration and partial purification of samples.

Figure 15 shows PAGE analysis of concentration of the feed stream with partial purification.

Figure 16 shows PAGE analysis of stream containing removed contaminants.

Figure 17 shows a second configuration of the present invention which allows for simultaneous concentration and purification of target protein with contaminant depletion of feed stream and EEO control of feed stream volume.

Figure 18 shows PAGE analysis of feed stream concentrated during the course of the experiment, but was also depleted of contaminant proteins. Contaminant depletion enhances the transfer of the target protein to the product stream by simplifying the contents of the feed stream.

Figure 19 shows PAGE analysis of target protein purified and concentrated into stream 2. The transfer rate to the product stream was maintained by concentrating the feed stream. As the concentration of target in the feed stream was depleted, the transfer rate slowed unless the feed stream volume was reduced. This volume reduction could result in interference from the increased contaminant concentration unless the feed stream is also depleted of contaminants.

Figure 20 shows a schematic diagram of a third configuration of present invention wherein the target is transferred to second stream and feed stream is concentrated.

Figure 21 shows a schematic diagram of a fourth configuration of the present invention which allows for the transfer of product to stream 1 while concentrating and optionally purifying stream 2 to the buffer stream or an optional third stream.

Figure 22 shows a schematic diagram of a fifth configuration of the present invention which uses EEO membranes on either side of the feed stream to enhance concentration effect, with MMCO of EEO membranes selected to allow for contaminant transfer away from the target protein which is retained and concentrated in the center feed stream.

Figure 23 shows a schematic diagram of a sixth configuration of the present invention which uses two membrane-based electrophoresis instruments for separate concentrating and purifying functions.

Figure 24 shows a schematic diagram of a seventh configuration of the present invention wherein the target remains in the feed stream and is concentrated.

Mode(s) for Carrying Out the Invention

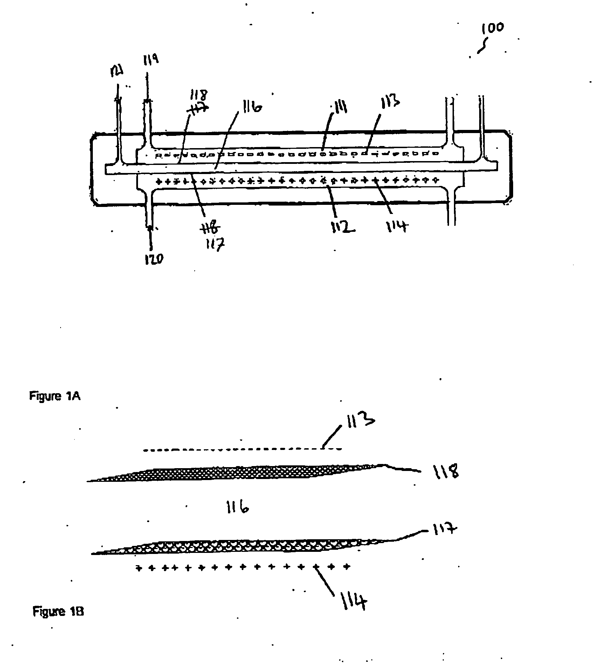

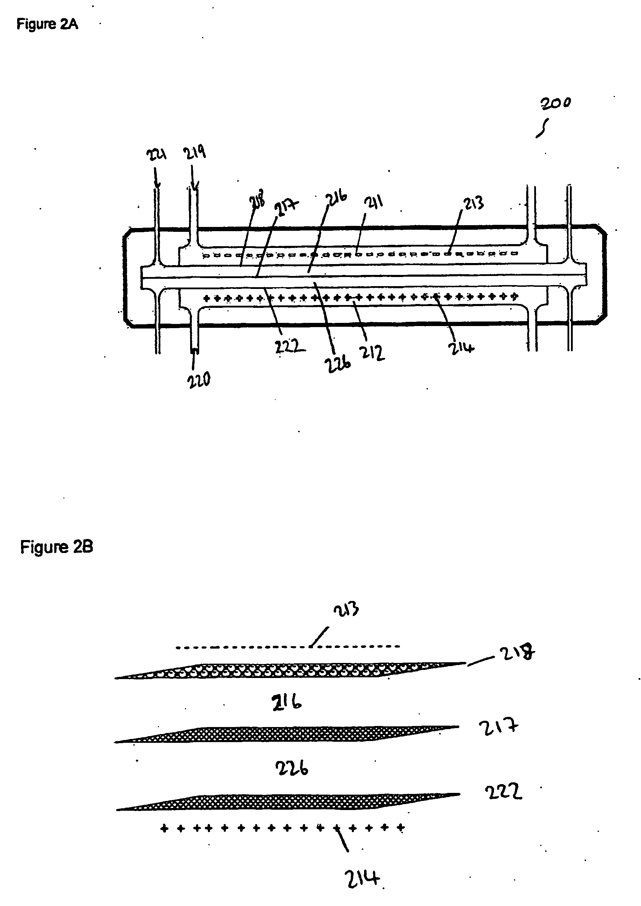

The present invention is directed to a membrane based electrophoresis technology to assist in the management of bulk liquid transfer/endo-osmosis. In one embodiment as shown in Figure 1A, the electrophoresis apparatus 100 is comprised of first and second electrode zones 111,112, wherein the first and second electrode zones 111,112 each zone contains an electrode 113, 114 and the second electrode zone 112 is disposed relative to the first electrode zone 111 so as to be adapted to generate an electric field in an electric field area 115 therebetween upon application of a selected electric potential between the electrodes 113, 114. The apparatus 100 is further comprised of a first interstitial volume 116 defined by a first membrane 117 disposed in the electric field area 115 and a second membrane 118 disposed between the first electrode zone 111 and the first membrane 117. The first interstitial volume 116 is separated from the first and second electrode zones 111,112 by the first and second membranes 117,118. At least one of the membranes is a barrier membrane capable of controlling substantial bulk movement of liquid under the influence of an electric field. In Figure 1B, the barrier membrane is depicted as the first membrane 117. It will be appreciated that the second membrane 118 can form the barrier membrane, In Figure 1, the first electrode 113 is depicted as a cathode and the second electrode 114 is depicted as an anode for convenience only. The polarity of the electrodes can be reversed where the first electrode 113 is an anode and the second electrode 114 is a cathode.

The apparatus 100 includes means 119,120,121 for communicating fluids to the first electrode zone 111, the second electrode zone 112, and the first interstitial volume 116 and at least one of the fluids contains a sample constituent. The apparatus 100 can further include means for applying a selected electric potential across at least the electric field area. The application of the selected electric potential causes at least one of at least a portion of any liquid within the sample constituent to migrate through at least one membrane into an adjacent electrode zone, and at least a portion of the sample constituent to migrate through at least one membrane into the first interstitial volume. The barrier membrane(s) controls substantial bulk movement of liquid into and out of the first interstitial volume such that a partially concentrated product is collected and/or remains in the first interstitial volume.

In use, a sample is placed in the first interstitial volume 116 (also called stream 1), buffer or solvent is provided to the electrode zones 111,112, an electric potential is applied to the electric field area causing movement of water out of the sample to the an adjacent electrode zone. The sample is thereby concentrated by driving liquid out of the sample. The barrier membrane substantially prevents bulk movement of liquid into the sample and the electrophoresis process causes water and salts to move out of the sample.

It is also feasible to place sample in one (or both) of the electrode zones and cause movement of one or more compounds from the sample into the adjacent interstitial volume during the application of the voltage potential.

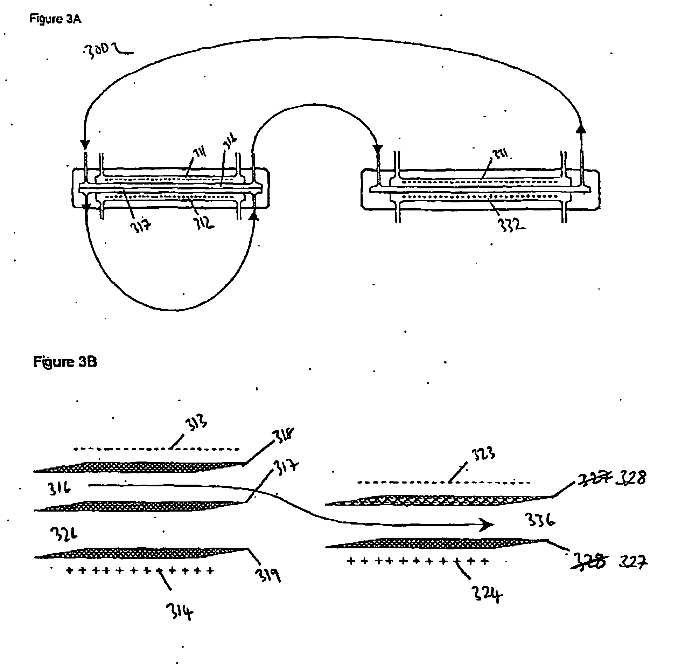

In another embodiment as shown in Figure 2A, the electrophoresis apparatus 200 is comprised of first and second electrode zones 211,212, wherein the first and second electrode zones 211,212 each contain an electrode 213,214. The second electrode zone 212 is disposed relative to the first electrode zone 211 so as to be adapted to generate an electric field in an electric field area 215 therebetween upon application of a selected electric potential between the electrodes 213,214. The apparatus 200 has first and second interstitial volumes 216,226. The first interstitial volume 216 is defined by a first membrane 217 disposed in the electric field area 215 and a second membrane 218 disposed between the first electrode zone 211 and the first membrane 217. The second interstitial volume 226 is defined by the first membrane 217 and a third membrane 222 disposed between the second electrode zone 212 and the first membrane 217. The first interstitial volume 216 is separated from the first electrode zone 211 by the second membrane 218 and the second interstitial volume 226 is separated from the second electrode zone 212 by the third membrane 222. At least one of the first, second, and third membranes 217,218,222 is a barrier membrane capable of controlling substantial bulk movement of liquid under the influence of an electric field. In Figure 28, the barrier membrane is depicted as the second membrane 218. It will be appreciated that the first membrane 217 or third membrane 222 can form the barrier membrane. In Figure 2, the first electrode 213 is depicted as a cathode and the second electrode 214 is depicted as an anode for convenience only. The polarity of the electrodes can be reversed where the first electrode 213 is an anode and the second electrode 214 is a cathode.

The apparatus includes means 219,220,221 for communicating fluids to the electrode zones 211,212 and interstitial volumes 216,226 and at least one of the fluids contains a sample constituent. The apparatus 200 can also include means for applying a selected electric potential across at least the electric field area. The application of the electric potential causes at least one of at least a portion of any liquid within the sample constituent to migrate through at least one membrane into an adjacent electrode zone, and at least a portion of the sample constituent to migrate through at least one membrane into at least one of the interstitial volumes. The barrier membrane(s) controls substantial bulk movement of liquid into and out of the interstitial volumes such that a partially concentrated product is collected and/or remains in at least one of the interstitial volumes.

In use, a sample is placed in the first interstitial volume (also called stream 1), buffer or solvent is provided to the electrode zones and the second interstitial volume (stream 2), an electric potential is applied to the electric field area causing at least one constituent in the sample to move to buffer/solvent in the adjacent electrode zone or to the adjacent second interstitial volume. The barrier membrane substantially prevents movement of liquid into the sample.

It will be appreciated that the order of interstitial volumes can be reversed where a sample is placed in the second interstitial volume (stream 2), buffer or solvent is provided to the electrode zones and the first interstitial volume, an electric potential is applied to the electric field area causing at least one constituent in the sample to move to the adjacent electrode zone or to the adjacent first interstitial volume (stream 1).

It is also feasible to place a sample in one (or both) of the electrode zones and cause movement of one or more compounds into one or more of the interstitial volumes during the application of the voltage potential.

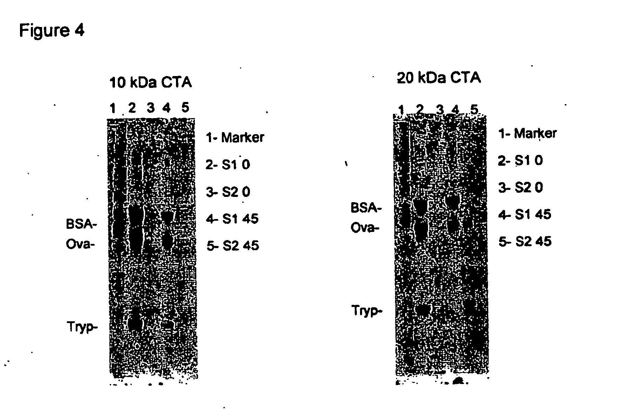

In another embodiment as shown in Figure 3, the electrophoresis apparatus 300 is comprised of first and second electrode zones 311,312, wherein the first and second electrode zones 311,312 each contain an electrode 313,314 and the second electrode zone 312 is disposed relative to the first electrode zone 311 so as to be adapted to generate an electric field in an electric field area therebetween upon application of a selected electric potential between the electrodes 313,314. The apparatus 300 has first and second interstitial volumes 316,326. The first interstitial volume 316 is defined by a first membrane 317 disposed in the electric field area and a second membrane 318 disposed between the first electrode zone 311 and the first membrane 317. The second interstitial volume 326 is defined by the first membrane 317 and a third membrane 319 disposed between the second electrode zone 312 and the first membrane 317. The first interstitial volume 316 is separated from the first electrode zone 311 by the second membrane 318. The second interstitial volume 326 is separated from the second electrode zone 312 by the third membrane 319.

The apparatus includes means for communicating fluids to the electrode zones and interstitial volumes and at least one of the fluids contains a sample constituent The apparatus also includes means for applying a selected electric potential across at least the electric field area. The application of the electric potential causes at least one of at least a portion of any liquid within the sample constituent to migrate through at least one membrane into an adjacent electrode zone, and at least a portion of the sample constituent to migrate through at least one membrane into at least one of the interstitial volumes. The barrier membrane(s) controls substantial bulk movement of liquid into and out of the interstitial volumes such that a partially concentrated product is collected and/or remains in at least one of the interstitial volumes.

The apparatus 300 further includes third 331 and fourth electrode zones 332, wherein the third and fourth electrode zones 331,332 each contain an electrode 323,324. The fourth electrode zone 332 is disposed relative to the third electrode zone 331 so as to be adapted to generate an electric field in an electric field area therebetween upon application of a selected electric potential between the electrodes. The apparatus 300 is further comprised of a third interstitial volume 336 defined by a fourth membrane 327 disposed in the electric field area and a fifth membrane 328 disposed between the third electrode zone 331 and the fourth membrane 327. The third interstitial volume 336 is separated from the third and fourth electrode zones 331,332 by the fourth and fifth membranes 327,328. At least one of the membranes is a barrier membrane capable of controlling substantial bulk movement of liquid under the influence of an electric field.

In Figure 3, the first electrode 313 is depicted as a cathode, the second electrode 314 is depicted as an anode, third electrode 323 is depicted as a cathode and the fourth electrode 324 as an anode for convenience only. The polarity of the electrodes can be reversed where the first and third electrodes 313,323 are anodes and the second and fourth electrodes 314,324 are cathodes. Similarly, the first and fourth electrodes 313,324 are anodes and the second and third electrodes 314,323 are cathodes.

Figure 3A shows the connection of two apparatus to form the apparatus according to the third aspect of the present invention. The first apparatus carries out some form of purification or separation of a sample while the second apparatus concentrates the partially purified sample. It is also feasible to have the first apparatus configured according to the second aspect of the present invention where at least one of the first, second or third membranes is also a barrier membrane capable of controlling substantial bulk movement of liquid under the influence of an electric field. In this form, the flow of fluid into or out of the sample can also be controlled.

The apparatus includes means for communicating fluids to the third electrode zone, the fourth electrode zone, and the third interstitial volume and at least one of the fluids contains a partially concentrated product from at least one of the first and second interstitial volumes. The apparatus further includes means for applying a selected electric potential across at least the electric field area. The application of the selected electric potential causes at least one of at least a portion of any liquid within the sample constituent to migrate through at least one membrane into an adjacent electrode zone, and at least a portion of the first partially concentrated product to migrate through at least one membrane into the third interstitial volume. The barrier membrane(s) controls substantial bulk movement of liquid into and out of the third interstitial volume such that a second partially concentrated product is collected and/or remains in the third interstitial volume.

EXPERIMENTAL AND RESULTS

The transfer of liquid from one area to another, through a porous membrane, is called endo-osmosis. Endo-osmosis is an issue with membrane-based electrophoresis technology and its management can increase product recovery, decrease run times and concentrate samples. One method to manage endo-osmosis is via electro-osmosis, where an external power source alters the rate of a system undergoing osmosis or endo-osmosis.

In-order to identify a biocompatible membrane, cellulose tri-acetate (CTA) membranes were used for a series of dialysis experiments where CTA was identified as a high endo-osmotic membrane. Due to the high endo-osmotic rate of CTA, its role in managing endo-osmosis was investigated. Additional work was also done to investigate the endo-osmotic rate of PVAI membranes.

The following parameters were investigated:

i) whether CTA endo-osmosis is voltage dependent;
ii) whether PVAI endo-osmosis is voltage dependent;
iii) whether CTA endo-osmosis is pH dependent; and
iv) whether CTA be incorporated into membrane-based electrophoresis technology to improve yield, purification rates and/or act as a concentrator.

**Materials**

Membrane-based electrophoresis apparatus made by Gradipore Limited, Australia

5 kDa, 500 kDa, 700 kDa and 1500 kDa molecular mass cut-off polyaccrylamide (PA) membranes (Gradipore)

5 kDa, 10 kDa and 20 kDa cellulose tri-acetate (CTA) membranes (Sartorius)

5% poly vinyl alcohol (PVAl) membrane (Gradipore Limited)

Bovine serum albumin (BSA) 67 kDa pI~5, ovalbumin 45 kDa pI 4.2, trypsin inhibitor 14 kDa pI 4.7, and fibrinogen 330 kDa pI 5.5

**Calibration of the molecular mass cut-off of CTA membranes**

The molecular mass cut off for the 10 kDa and 20 kDa CTA membranes were determined for use in membrane-based electrophoresis technology. The 10 kDa and 20 kDa CTA membranes were used in separate purification runs where 3 mg/mL protein mixture (1 mg/mL of each - BSA, ovalbumin and trypsin inhibitor in TB pH 8.0) was placed in the stream 1 and separated with 250V for 45 min into the stream 2. A non-reduced PAGE of the 0 min stream 1 and 45 min stream 2 were run to determine the molecular mass cut-off of the 10 kDa and 20 kDa CTA membranes (Figure 4).
The results of the CTA calibration experiments (Figure 4), suggested that the actual cut-off for the CTA membranes were 14 kDa for the 10 kDa membrane and 45 kDa for the 20 kDa CTA membrane. The 14 kDa molecular mass cut off of the 10 kDa CTA membrane was determined from the PAGE as only trypsin inhibitor (14 kDa) was observed after passing through the 10 kDa CTA membrane during the 45 min electrophoresis run. The 45 kDa molecular mass cut off of the 20 kDa CTA membrane was determined from the PAGE as trypsin inhibitor (14 kDa), ovalbumin (45 kDa) and a small quantity of BSA (67 kDa) passed through the 20 kDa CTA membrane during the 45 min electrophoresis run.

**Orientation of CTA membranes**

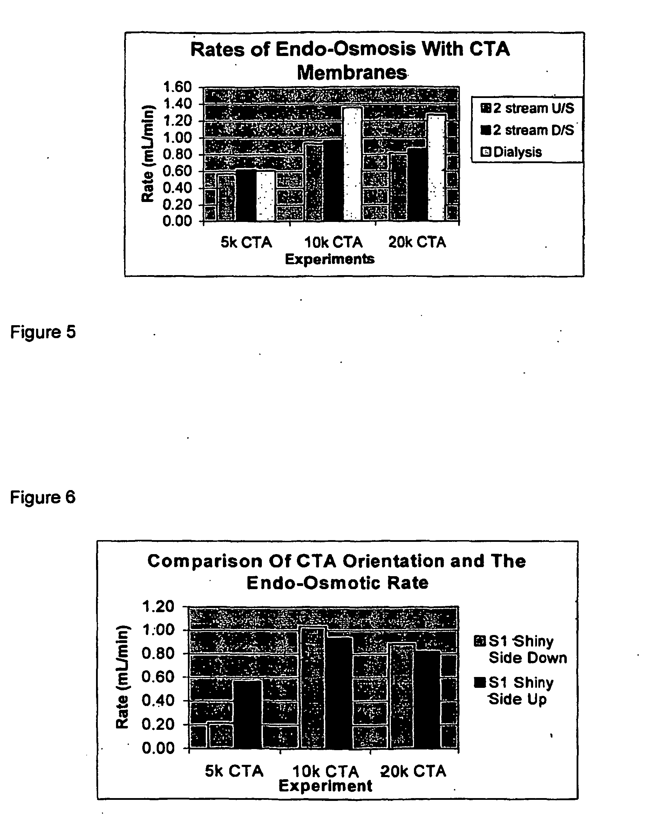

CTA membranes were asymmetric, therefore to use CTA membranes for further experimentation, the orientation (shiny side up or down) and endo-osmotic rate of 5 kDa, 10 kDa and 20 kDa CTA membranes were analysed. These parameters were investigated in two stream and single stream apparatus configurations in order to identify which configuration had the highest endo-osmotic rate. This involved recording volume variations every 5 min and comparing the amount of starting BSA (1 mg/mL) to the final amount of BSA.
The experimental data suggested that the 5 kDa CTA membrane had the slowest endo-osmotic rate in both the standard and single steam configuration, compared to the other CTA membranes (Figure 5). The endo-osmotic rates for both the 10 kDa and 20 kDa membranes were comparably high, with the single stream configuration demonstrating a higher endo-osmotic rate than the standard configuration (Figure 5). The 10 kDa CTA membrane had the highest endo-osmotic rate in both configurations compared to the 5 kDa and 20 kDa CTA membranes.
As the CTA membranes were asymmetric (a shiny side and a dull side) a comparison of the orientation was also conducted. The results indicated that the endo-osmotic rate of the shiny side up 5 kDa CTA membrane was higher than the shiny side down (Figure 6). However, for the 10 kDa and 20 kDa CTA membranes shiny side down performed slightly better than the shiny side up. Overall, the 10 kDa CTA membrane had a higher endo-osmotic rate.
In conclusion, single stream configuration of membrane-based electrophoresis technology using a 10 kDa CTA membrane, in either symmetry, produced the highest endo-osmotic rate. This provided the foundation for the remaining experimental procedures using CTA membranes. For consistency, all remaining CTA experiments were single stream configuration, using a 10 kDa shiny side up membrane.

**Endo-osmosis and voltage using CTA membranes**

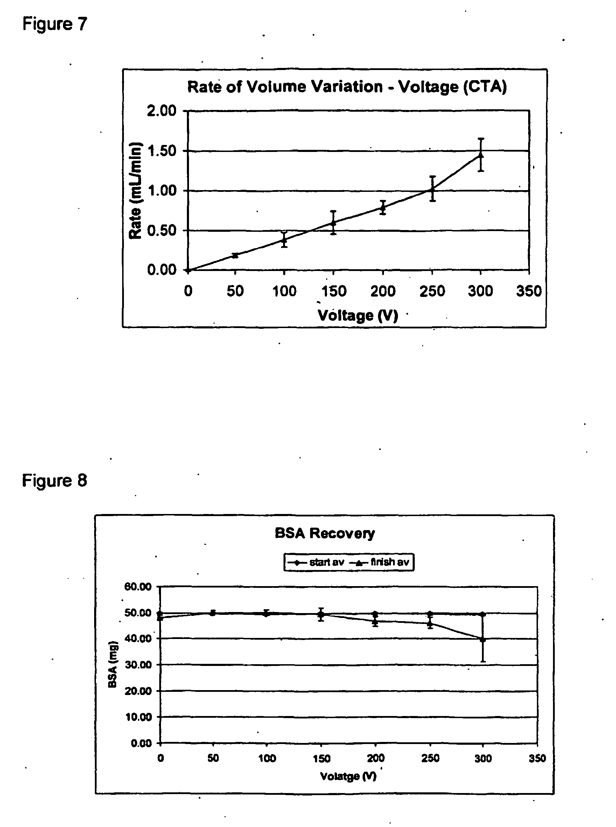

To determine whether endo-osmosis was voltage dependent, a series of experiments in which the volume change due to electro-osmosis were prepared. To investigate whether CTA endo-osmosis was voltage dependent, an electrophoresis apparatus was set-up in single stream configuration, with cooled TB pH 9.0 in the buffer tank, and a cartridge consisting of a polyacrylamide (PA) restriction membrane and a top CTA separating and, or volume control barrier membrane. Fifty mL of 1 mg/mL BSA was run through the cartridge in the stream 2 of the apparatus for 30 min. The change in volume was measured every 5 min and the concentration of the initial and final stream 2 were determined by spectrophotometry ($A_{280}$). The above experimental set-up was repeated from 0V to 300V (1A, 300W) at 50V increments.
The experimental data showed that as the voltage increased, the rate of volume loss increased linearly (Figure 7). The results suggested that the endo-osmotic rate, in conjunction with CTA as a volume control barrier , can be managed by altering the voltage for procedures in which bulk liquid transfer is an issue. However, as the voltage increased, the amount of BSA recovered was reduced (Figure 8). The reduced recovery of BSA was attributed to the increased voltage forcing the BSA into the restriction PA membrane.

### Endo-Osmosis and voltage using PVAI Membranes

To determine whether endo-osmosis was voltage dependent using PVAI membranes, experiments were carried out using the method used for CTA membranes outlined in the section above.

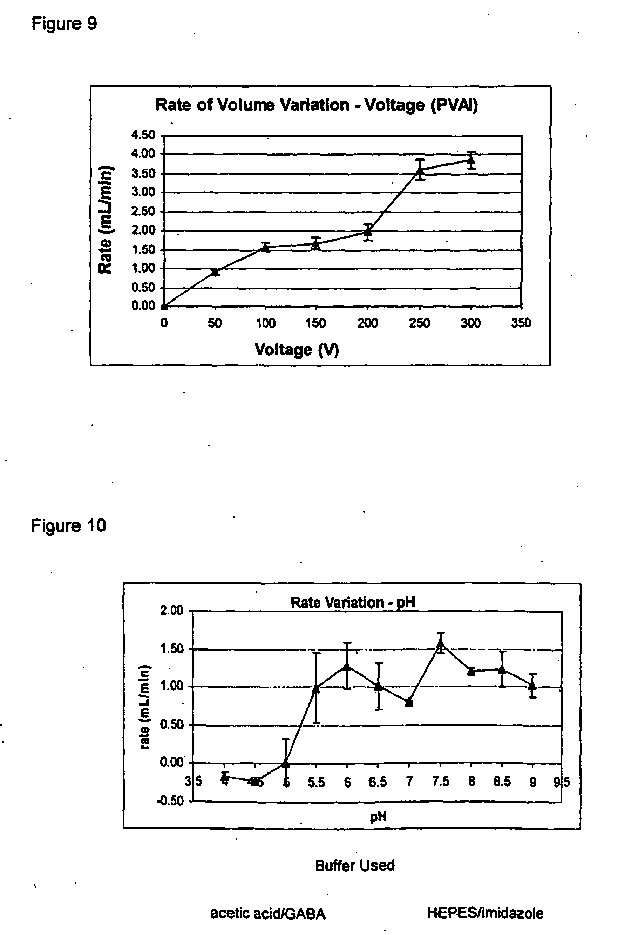

The experimental data showed that as the voltage was increased, the rate of volume reduction also increased (Figure 9). However, the correlation was not as linear as that achieved using the CTA membranes. Comparison of CTA and PVAI electro-endo-osmosis rates using 200V on an apparatus in single stream configuration with BSA, resulted in a 4x increase in the electro-endo-osmosis rate when using PVAI, compared to CTA. There was no change in the electro-endo-osmosis rate when using PA membranes.

### Endo-Osmosis and pH using CTA membranes

To determine whether endo-osmosis was pH dependent, a series of experiments in which the volume reduction due to electro-endo-osmosis were compared. To investigate whether CTA endo-osmosis was pH dependent an electrophoresis apparatus was set-up in single stream configuration, with cooled TB pH 9.0 in the buffer tank, and a cartridge consisting of a bottom PA membrane restriction and a top CTA separating and, or volume control barrier membrane. Fifty mL of 1 mg/mL BSA was run through the cartridge in the stream 2 of the apparatus for 30 min at 250V (1A, 300W). The change in volume was measured every 5 min and the concentration of the initial and final stream 2 were determined by spectrophotometry ($A_{280}$). The above experimental set-up was repeated from pH 4.0 to pH 9.0, at pH 0.5 increments.

The experimental data suggested that as the pH increased, the rate of volume reduction increased (Figure 10). However, each buffer had its own variations, suggesting that pH dependent endo-osmosis rates may need to be determined for individual applications and buffer systems.

### Endo-Osmosis management and isolation of fibrinogen

To test whether the endo-osmotic rate could be managed with a charged membrane, a procedure with a high endo-osmotic rate was studied. The procedure with a high endo-osmotic rate was the isolation of fibrinogen from cryo-precipitate. Isolating fibrinogen from cryo-precipitate typically resulted in the increase of the stream 1 volume by a factor of 5 to 8, with fluid being drawn from the stream 2 during the purification process. In order to test the effect of altering the accumulation of fluid in stream 1, fibrinogen was isolated from a stock cryo-precipitate (1:3 dilution cryo-precipitate stock solution was prepared and used as the starting material for all three methods), using four different methods.

Method 1: Standard isolation of fibrinogen using electrophoresis apparatus with a 700-1500-700 kDa PA membrane cartridge, at 250V (1A, 300W). The buffer tank contained cooled TB pH 9.0, 30 mL 1:3 diluted cryo-precipitate stock solution was placed in stream 1, and 10 mL TB pH 9.0 in stream 2. The change in stream 1 volume was noted every 15 min, along with a spectrophotometry ($OD_{280}$) reading of the stream 2. Stream 2 was harvested every 30 min, the buffer replaced and the voltage reversed for 2 min (to de-foul the membrane).

Method 2: Similar to method 1, with the exception that the top restriction membrane (700 kDa PA membrane) was replaced with a 10 kDa CTA volume control barrier membrane.

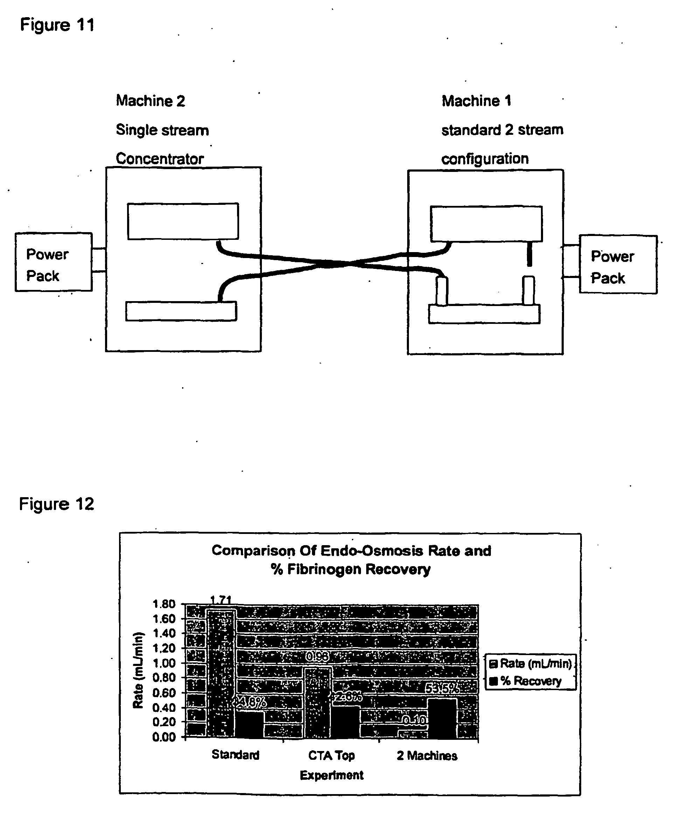

Method 3: Two membrane-based electrophoresis apparatus were used, with separate power supplies, attached together as shown in Figure 11. Apparatus 1 was prepared and run as described in method 1 (note: the 700 kDa restriction membranes were replaced with 500 kDa restriction membranes), apparatus 2, however, was configured as a single stream with a 5 kDa PA membrane (bottom) 10 kDa CTA membrane (top). Apparatus 2 was connected to stream 1 of apparatus 1 and functioned as a stream 1 concentrator, by managing the endo-osmotic rate with the separate power supply. From the data obtained from the voltage dependence experiments above, a voltage which matched the endo-osmotic rate of method 1 was chosen. The voltage of apparatus 2 was 250V (1A, 300W).

Method 4: Similar to method 1, with the exception that the top restriction membrane was replaced with a PVAI membrane.

Using CTA during fibrinogen isolation was found to decrease the amount of endo-osmosis (Figure 12) by a factor of 2 (method 2) to a factor of 6 (method 3). Using a separate power supply and apparatus with a concentrator cartridge, the stream 1 endo-osmosis rate was maintained at a low endo-osmotic rate, without altering voltage (voltage was set at a constant 250V). Unlike method 3, stream 2 volume of methods 1 and 2 required continual monitoring and toping up.

Method 1 isolated 34.8% fibrinogen, while method 2 isolated 42.6% fibrinogen and method 3 isolated 53.5% fibrinogen from a stock cryo-precipitate solution (Figure 12). Overall, method 2 and 3 appeared to be commercially suitable due to the high percentage recovery and decrease of the endo-osmosis rate.

The use of PVAI during a fibrinogen isolation resulted in a 4x increase in the protein transfer rate in the apparatus (Table 1). Using PVAI as a top restriction membrane, to reduce electro-endo-osmosis, resulted in an increase in protein transfer from 44.7% to 77.3%. These results also demonstrate using PVAI can decrease or improve the recovery time. When using PA membranes, 32.5 mg fibrinogen was recovered after 120 min, while 36.1 mg of fibrinogen was recovered in 30 min using PVAI. Using PVAI therefore allowed more fibrinogen to be removed in a quarter of the time.

Table 1. Comparison of protein transfer during fibrinogen isolation. The table compares using PAM and PVAI during fibrinogen isolation.

| | Control | PVAI top membrane |
|---|---|---|
| **Total protein recovered** | 24.3% | 31.3% |
| **Total protein recovered after 30 min** | 44.7% | 77.3% |
| **Recovery comparison** | 32.5 mg/120 min (0.27 mg/min) | 36.1 mg/30 min (1.20 mg/min) |
| **Electro-endo-osmosis (mL / min)** | 1.71 | 0.00 |

**Simultaneous separation and concentration of bovine Prion Protein (PrP) of bovine brain homogenate using PVAI membrane**

Another application for membrane-based electrophoresis involved the simultaneous separation and concentration of the bovine Prion Protein (PrP) of bovine brain homogenate, using a PVAI membrane as a top restriction membrane separating stream 1 from the first electrode zone. Typically during separation experiments involving PrP, electro-endo-osmosis causes an increase in the volume of the starting material. However, with the introduction of PVAI as a top restriction membrane, the electro-endo-osmosis rate was decreased.

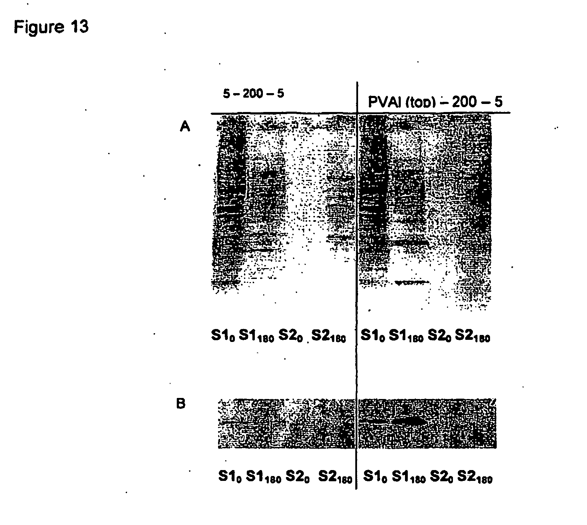

In Figure 13, a comparison of a typical PrP separation and a separation & concentration of PrP using PVAI has been provided. Part A of Figure 13, an SDS-PAGE of the samples from the two electrophoresis experiments, demonstrates transfer of protein from stream 1 to stream 2. While part B of Figure 13 is the corresponding western blot of the samples hybridised with anti-PrP R029 (Prionics, Switzerland). Normally, separation of PrP is carried out for 3 hours at 250V using a cartridge with a separation membrane of 200 kDa, and two restriction membranes of 5 kDa using Tris Borate buffer pH 9.0 (Part A, lanes 1-4), the conditions under which PrP remained in stream 1 (Part B, lanes 1-4). When using the same conditions, but replacing the top restriction membrane with PVAI membrane, simultaneous separation and concentration of PrP from bovine brain homogenate was achieved.

The use of PVAI resulted in a 5x decrease in the volume of the stream 1 at the end of the experiment and thus a stark increase in the concentration of PrP, as detected by the increase of intensity of the PrP band on the Western blot (Part B).

CONCLUSION

The highest endo-osmotic rate was produced using a single stream configuration electrophoresis apparatus with a 10 kDa CTA membrane, in either symmetry. The identification of the configuration with the highest endo-osmotic rate allowed for experiments to manage this rate. Analysing whether endo-osmosis was voltage dependent demonstrated that the rate of volume loss linearly increased as the voltage increased. Therefore, increasing the voltage of a system can reduce the effect of endo-osmosis, by electro-osmosis. The relationship between pH and endo-osmosis suggest that each buffer system had its own variables and that pH may need to be investigated on an individual application basis to obtain optimum separation and / or concentration.

To test whether the endo-osmotic rate could be managed with a charged membrane (CTA), a procedure with a high endo-osmotic rate was studied. The procedure with a high endo-osmotic rate was the isolation of fibrinogen from cryo-precipitate. Three experiments were used to study endo-osmosis management. The first experiment was a control which identified the endo-osmosis rate of a standard fibrinogen isolation run. The second experiment involved reducing the endo-osmotic rate by replacing the top PA membrane restriction with CTA. The final experiment utilised a second apparatus, in a single stream configuration (CTA top and PA membrane on the bottom), which managed the-stream 1 volume of the first apparatus. Each apparatus had its own power supply. Therefore the rate of electro-osmosis in apparatus 2 could be managed by varying the voltage (matching the electro-osmotic rate). The results of these experiments demonstrated that endo-osmotic rate of the stream 1 could be managed thereby increasing total recovery of fibrinogen and accelerating purification.

The control of the endo-osmotic rate also has application in investigations involving PrP. The use of PVAI demonstrates a method for the simultaneous concentration and separation of PrP. This method has applications in product purification along with prion detection and diagnostics.

**Electro endo-osmosis apparatus configurations for monoclonal antibody/recombinant purifications/concentration**

The present invention is especially applicable to simultaneous purification and concentration of proteins which are grown by recombinant or tissue culture means and are produced in a dilute form in large liquid volumes. Use of the method and apparatus according to the present invention allows for numerous configurations where simultaneous purification and concentration/volume control can take place.

Purification procedures may take one of two general forms:

- transfer of target protein away from contaminants which are retained in the feed stream (sample)

- transfer of contaminants away from the target protein which is retained in the feed stream (sample)

In the first form, as the target protein concentration falls, the rate of target protein transfer will also fall, resulting in slower purification. The contribution of endo-osmosis may also increase the feed stream volume, further slowing purification. The ability to concentrate or control the volume of the feed stream allows the transfer of target protein to be maintained at the highest possible rate.

In the second form, as the contaminant concentration in the feed stream falls, purification will also slow, prolonging purification time. The ability to concentrate the feed stream where product is accumulating will enhance the rate of purification by maintaining the transfer rate of contaminants from the target protein, simultaneously delivering a concentrated target protein.

Both these techniques are applicable with respect to any protein (or other compound) that is present in low concentrations in the feed stream, especially recombinant and monoclonal antibody (MAb) proteins which are routinely grown at concentrations between 5 and 100 $\mu$g/mL in tissue culture medium.

Several configurations of the membrane-based electrophoresis apparatus can be used to effect simultaneous purification and concentration of a product that is present at low concentrations in a large volume.

APPLICATIONS

**Configuration 1**

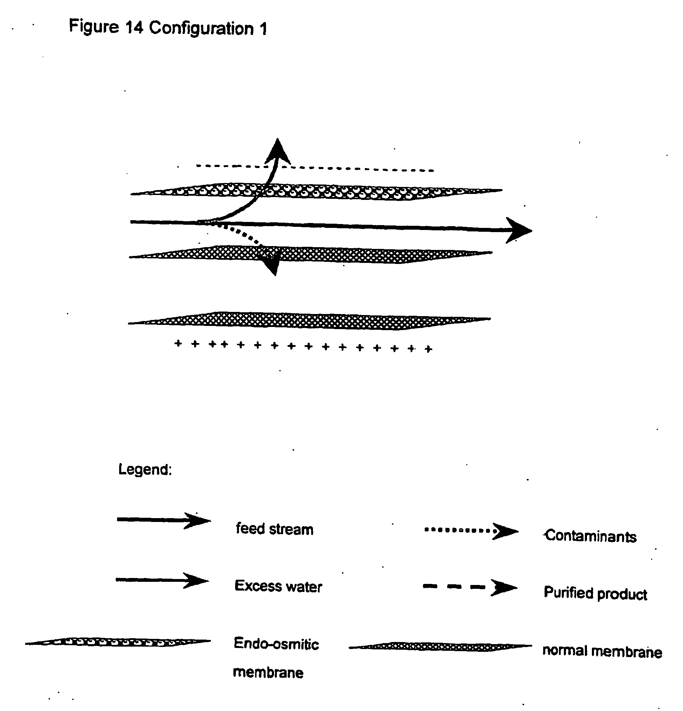

Simultaneous concentration and partial purification (Figure 14).

In a single apparatus having one sample stream (stream 1) and a separation stream (stream 2), a top restriction membrane with electro-endo-osmosis properties is used to draw water from the large volume of dilute feed stream. Conditions are chosen such that some, or ideally all contaminants are transferred to the second stream while the target protein (and possibly some remaining contaminants) are concentrated by electro-endo-osmosis in the feed stream.

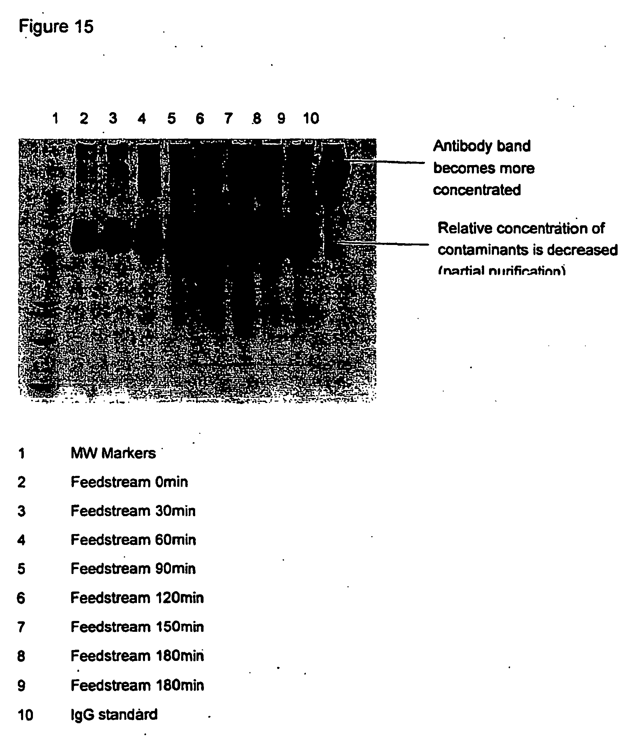

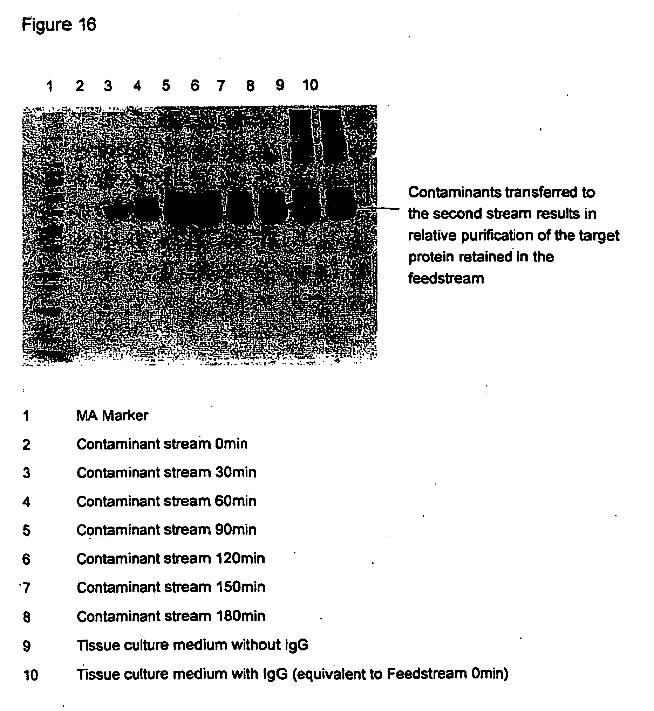

In an example of this method, a 130 mL feed stream was concentrated to 10 mL (13 fold concentration) in 180 minutes, while significant quantities of albumin and transferrin contaminants were removed to the second stream. Results of PAGE analysis are shown in Figure 15 and Figure 16. This example provided partial purification of the target and significant concentration of the product. The example provided was carried out with tissue culture supernatant containing 10% FCS. This configuration is even more suitable for serum-free medium where the target protein is a significant proportion of the total protein. This method could be improved with alterations to the separation membrane and buffer conditions, as well as the cut off of the electro-endo-osmosis membrane.

Antibody concentration was increased over 10-fold during a 3 hour period. Relative concentration of contaminants was decreased over this time by transferring contaminants to a second stream (Figure 16).

The data in Figure 15 and Figure 16 show that contaminants were selectively removed from the feed stream without loss of the target protein, indicating partial purification of the target protein.

**Configuration 2**

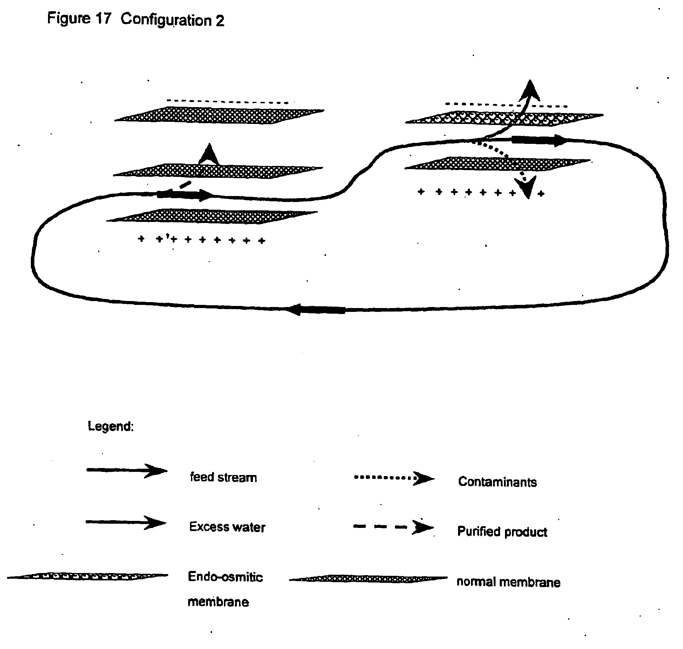

Simultaneous concentration and purification of target protein with contaminant depletion of feed stream and electro-endo-osmosis control of feed stream volume (Figure 17).

Using two apparatus, one apparatus was configured to transfer target protein into a separate product stream away from contaminants that were retained in the feed stream. In a separate apparatus, a cartridge using an electro-endo-osmosis top restriction membrane was used to concentrate the feed stream to maintain the transfer rate of target protein in the first separation unit. In the example given below, the second apparatus was also configured to allow transfer of contaminants from the feed stream into the buffer stream of the second instrument, thereby depleting the feed stream of contaminants.

In an example of this method, a 130 mL feed stream was concentrated to 100 mL over a period of 180 minutes, while albumin and transferrin contaminants were removed to the buffer stream of the second apparatus. This provided excellent purification of the target protein, significant concentration of the product by transferring the product to a small stream volume, but also allowed for cleaning and concentrating the feed stream to enhance target protein transfer. The example provided was carried out with tissue culture supernatant containing 10% fetal calf serum(FCS). This method could be enhanced through the use of a electro-endo-osmosis membrane capable of greater liquid transfer, or the use of an electro-endo-osmosis membrane capable of drawing liquid towards the positive electrode.

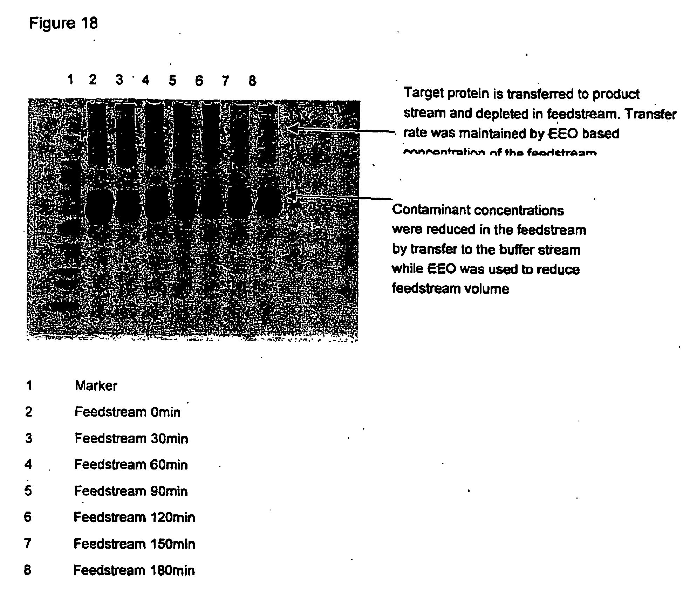

Figure 18 shows PAGE analysis of results the above experiment. Feed stream was concentrated during the course of the experiment, but was also depleted of contaminant proteins. Contaminant depletion enhances the transfer of the target protein to the product stream by simplifying the contents of the feed stream.

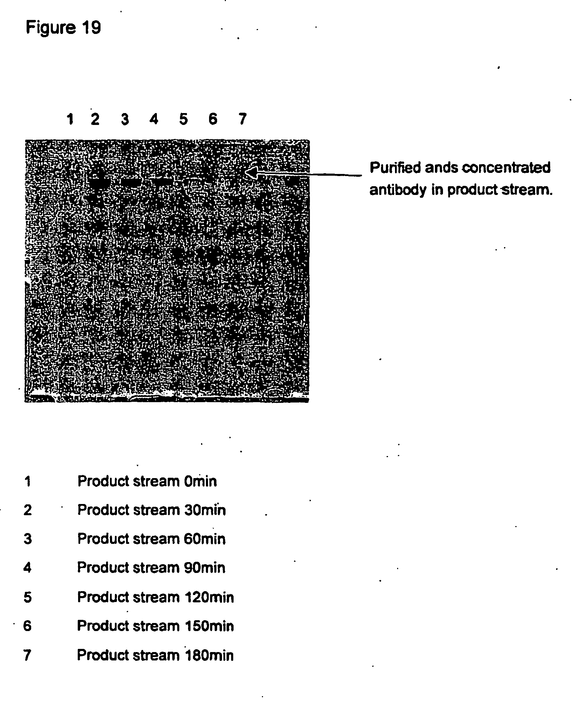

Figure 19 shows PAGE of final purification results the above experiment. The target protein was purified and concentrated into the product stream. The transfer rate to the product stream was maintained by concentrating the feed stream. As the concentration of target in the feed stream was depleted, the transfer rate slowed unless the feed stream volume was reduced. This volume reduction would result in interference from the increased contaminant concentration unless the feed stream was also depleted of contaminants.

Alternative configurations where electro-endo-osmosis can be used to purify and concentrate target proteins from dilute starting materials.

**Configuration 3**

Target is transferred to second stream and feed stream is concentrated.

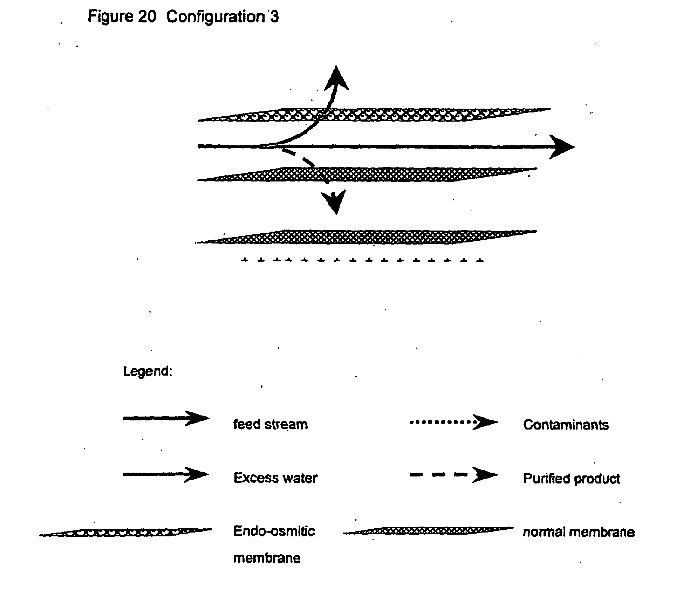

A single apparatus configured as shown in Figure 20, having one sample stream (stream 1) containing a sample to be treated and a separation stream (stream 2) into which a selected compound is transferred by electrophoresis. A top restriction membrane with electro-endo-osmosis properties is used to draw water from the sample stream. Conditions are chosen such that some, or ideally all of a selected compound is transferred to the second stream while the sample is concentrated by electro-endo-osmosis in the feed stream to maintain flux rate of product stream.

**Configuration 4**

Transfer of product to stream 1 while concentrating and optionally purifying stream 2 with contaminants transferred to the buffer stream or an optional third stream.

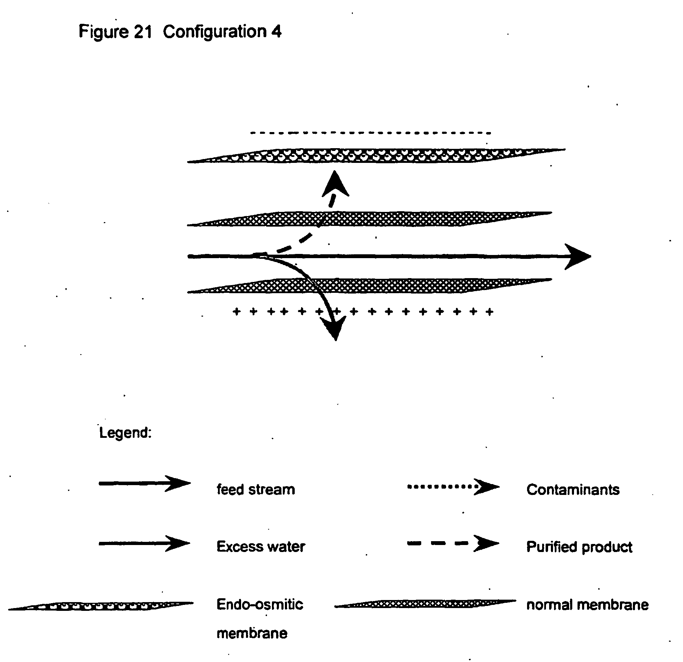

A single apparatus configured as shown in Figure 21, having one sample stream (stream 1) containing a sample to be treated and a separation stream (stream 2) into which a selected compound is transferred by electrophoresis. A top restriction membrane with electro-endo-osmosis properties is used to drive water from the sample stream through the third membrane. Conditions are chosen such that some, or ideally all of a selected compound is transferred to the second stream while the sample is concentrated by electro-endo-osmosis in the feed stream to maintain flux rate of compound in the sample stream.

**Configuration 5**

Use of electro-endo-osmosis membranes on either side of the feed stream to enhance concentration effect, with molecular mass cut-off of electro-endo-osmosis membranes selected to allow for contaminant transfer away from the target protein which is retained and concentrated in the center feed stream.

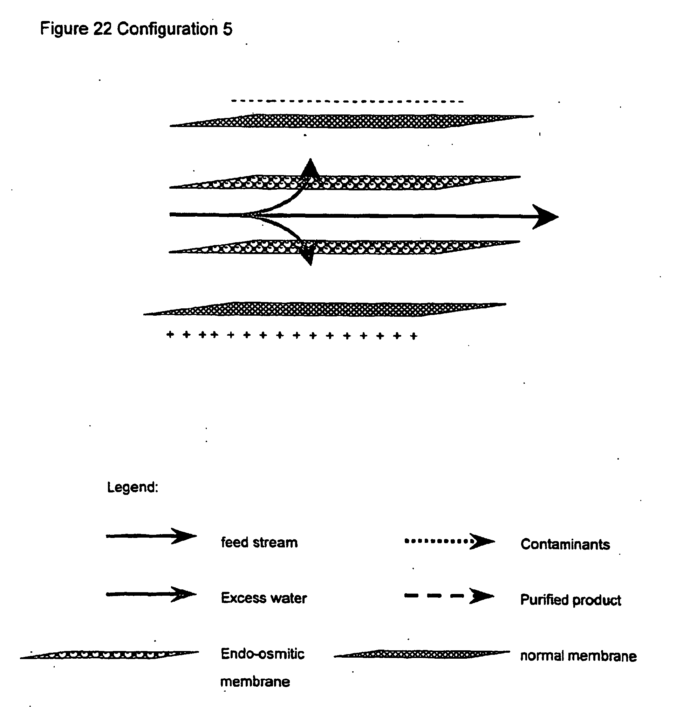

A single apparatus configured as shown in Figure 22, having one sample stream (stream 1) containing a sample to be concentrated and cleaned and upper and lower stream (streams 2 and 3) into which water and unwanted contaminants are transferred by electrophoresis. Two restriction membranes with electro-endo-osmosis properties form the sample stream and are used to drive water from the sample stream through to the upper and lower streams. Conditions are chosen such that some, or ideally all contaminants are transferred to the second or third streams while the sample is concentrated by electro-endo-osmosis in the feed stream.

**Configuration 6**

Use of two electrophoresis apparatus for separate concentrating and purifying functions.

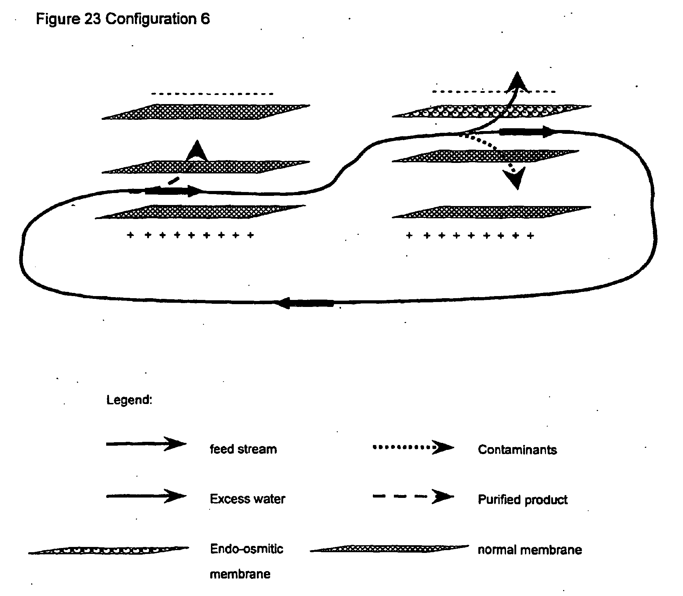

This example uses two apparatus as shown in Figure 23. One apparatus is configured to transfer target protein into a separate product stream away from contaminants that were retained in the sample stream. In a separate apparatus, a cartridge using an electro-endo-osmosis top restriction membrane and two other membranes forming two streams to concentrate the sample stream, remove contaminants and to maintain the transfer rate of target protein in the first separation unit.

**Configuration 7**

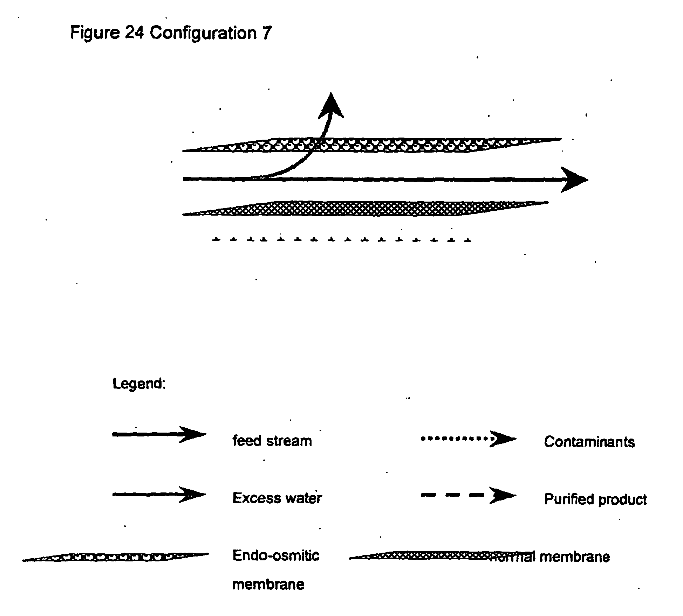

A single apparatus configured as shown in Figure 24, having one sample stream (stream 1) containing a sample to be concentrated and cleaned. One restriction membrane with electro-endo-osmosis properties and is used to drive water from the sample stream through to an electrode zone to form a concentrated sample.

Many other configurations are possible depending on target protein and feed stream contaminant profiles, using different membrane chemistry and buffer combinations that draw bulk liquid in the required directions and molecular mass cut-off of membranes tuned for optimal transfer of contaminants or target proteins.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

**Claims**

1. An electrophoresis apparatus (100) comprising:

a first electrode (113) in a first electrode zone (111);
a second electrode (114) in a second electrode zone (112), the first electrode (113) disposed relative to the second electrode (124) so as to be adapted to generate an electric field in an electric field area therebetween upon application of an electric potential between the first and second electrodes (113, 114);
a first membrane (117) disposed in the electric field area;
a second membrane (118) disposed between the first electrode zone (111) and the first membrane (117) so as to define a first interstitial volume (116) therebetween, wherein the first interstitial volume (116) is separated from the first and second electrode zones (111, 112) by the first and second membranes (117, 118);
means (119, 120) adapted to communicate liquid to the first electrode zone (111) and the second electrode zone (112); and
means (121) adapted to communicate a sample constituent to the first interstitial volume (116);

**characterised in that** at least one membrane is a barrier in the form of an inducible electro-endo-osmotic membrane capable of controlling substantial bulk movement of liquid under the influence of an electric field, wherein the at least one inducible electro-endo-osmotic membrane is either

(a) made from cellulose triacetate with a molecular mass cut-off from 5 to 20 kDa, or
(b) made from polyvinyl alcohol.

2. The apparatus (200) of claim 1, further comprising:

a third membrane (222) disposed between a second electrode zone (212) and the first membrane (217) so as to define a second interstitial volume (226) therebetween, wherein the first interstitial volume (216) is separated from the first electrode zone (211) by the second membrane (218) and the second interstitial volume (226) is separated from the second electrode zone (212) by the third membrane (222).

3. The apparatus (300) of claim 2, further comprising:

a third electrode (323) in a third electrode zone (331);
a fourth electrode (324) in a fourth electrode zone (332), the third electrode (323) disposed relative to the fourth electrode (324) so as to be adapted to generate an electric field in a second electric field area therebetween upon application of a second electric potential between the third and fourth electrodes (323, 324);
a fourth membrane (327) disposed in the second electric field area;
a fifth membrane (328) disposed between the third electrode zone (331) and the fourth membrane (327) so as to define a third interstitial volume (336) therebetween, wherein the third interstitial volume (336) is separated from the third and fourth electrode zones (331, 332) by the fourth and fifth membranes (327, 328), and wherein at least one of the fourth or fifth membranes (327, 328) is a barrier capable of controlling substantial bulk movement of liquid under the influence of an electric field;
means adapted to communicate liquid to the third electrode zone (331) and the fourth electrode zone (332); and
means adapted to communicate a sample constituent from the first or second interstitial volume (316, 326) to the third interstitial volume (336).

4. The apparatus of any one of claims 1 to 3, further comprising:

means adapted for removing heat generated in the apparatus.

5. The apparatus of any one of claims 1 to 4, wherein the inducible electro-endo-osmotic membrane has a defined pore size.

6. The apparatus of claim 5, wherein the inducible electro-endo-osmotic membrane is made from polyvinyl alcohol and has a defined pore size from about 5 to about 1000 kDa.

7. The apparatus of any one of claims 1 to 6, wherein the inducible electro-endo-osmotic membrane is adjacent to one of the electrode zones.

8. The apparatus of any one of claims 1 to 7, wherein one or more membranes are electrophoresis separation membranes having defined pore sizes.

9. The apparatus of claim 8, wherein the electrophoresis separation membranes are made from polyacrylamide.

10. The apparatus of claim 9, wherein the electrophoresis separation membranes have defined pore sizes from about 1 to about 2000 kDa.

11. A method for concentrating a sample, the method comprising:

(a) providing an electrophoresis apparatus (100) according to any one of claims 1 to 10;
(b) adding buffer or solvent to the first and second electrode zones (111, 112);
(c) adding buffer or solvent to the third and fourth electrode zones, if present;
(d) adding the sample to the first interstitial volume (116);
(e) applying an electric potential between the electrodes (113, 114) causing liquid in the sample to move through a membrane into an adjacent electrode zone (111, 112); and
(f) optionally obtaining the concentrated sample;

wherein the barrier membrane allows bulk movement of liquid out of the sample.

12. A method for moving at least one component from a sample while controlling the bulk movement of liquid, the method comprising:

(a) providing an electrophoresis apparatus (200) according to claim 2 or any one of claims 3 to 10 when dependent on claim 2;
(b) adding buffer or solvent to the first and second electrode zones (211, 212);
(c) adding buffer or solvent to the third and fourth electrode zones, if present;
(d) adding the sample to the first interstitial volume (216);
(e) adding buffer or liquid to the second interstitial volume (226);
(f) applying an electric potential between the electrodes (213, 214) causing at least one compound from the sample to move through a membrane into an adjacent electrode zone or interstitial volume; and
(g) optionally collecting the compound moved from the sample;

wherein the barrier membrane controls substantial bulk movement of liquid into or out of one or more of the interstitial volumes.

13. A method for moving at least one component from a sample While controlling the bulk movement of liquid, the method comprising:

(a) providing an electrophoresis apparatus (300) according to claim 3 or any one of claims 4 to 10 when dependent on claim 3;

(b) adding buffer or electrolyte to the first, second, third and fourth electrode zones (311, 312, 331, 332);

(c) adding the sample to the first interstitial volume (316);

(d) adding buffer or liquid to the second interstitial volume (326);

(e) applying a first electric potential between the first and second electrodes (313, 314) causing at least one compound from the sample to move through a membrane into the second interstitial volume (326);

(f) passing sample containing the compound from either the first or second interstitial volume (316, 326) to the third interstitial volume (336);

(g) applying a second electric potential between the third and fourth electrodes (323, 324) causing liquid in the third interstitial volume (336) to move through a membrane into an adjacent electrode zone (331, 332) thereby concentrating the compound in the sample; and

(h) optionally collecting the concentrated sample;

wherein the barrier membrane controls substantial bulk movement of liquid into the third interstitial volume (336).

14. An electrophoresis process comprising the steps of:

(a) providing an apparatus (100) comprising:

a first electrode (113) in a first electrode zone (111);
a second electrode (114) in a second electrode zone (112), the first electrode (113) disposed relative to the second electrode (114) so as to be adapted to generate an electric field in an electric field area therebetween upon application of an electric potential between the first and second electrodes (113, 114);
a first membrane (117) disposed in the electric field area;
a second membrane (118) disposed between the first electrode zone (111) and the first membrane (117) so as to define a first interstitial volume (116) therebetween, wherein the first interstitial volume (116) is separated from the first and second electrode zones (111, 112) by the first and second membranes (117, 118), and wherein at least one membrane is a barrier in the form of an inducible electro-endo-osmotic membrane capable of controlling substantial bulk movement of liquid under the influence of an electric field;
means (119, 120) adapted to communicate liquid to the first electrode zone (111) and the second electrode zone (112); and
means (121) adapted to communicate a sample constituent to the first interstitial volume (116); and

(b) controlling substantial bulk movement of liquid under the influence of an electric field with the at least one inducible electro-endo-osmotic membrane.

15. The process of claim 14, wherein the apparatus (200) further comprises:

a third membrane (222) disposed between a second electrode zone (212) and the first membrane (217) so as to define a second interstitial volume (226) therebetween, wherein the first interstitial volume (216) is separated from the first electrode zone (211) by the second membrane (218) and the second interstitial volume (226) is separated from the second electrode zone (212) by the third membrane (222).

16. The process of claim 15, wherein the apparatus (300) further comprises:

a third electrode (323) in a third electrode zone (331);
a fourth electrode (324) in a fourth electrode zone (332), the third electrode (323) disposed relative to the fourth electrode (324) so as to be adapted to generate an electric field in a second electric field area therebetween upon application of a second electric potential between the third and fourth electrodes (323, 324);
a fourth membrane (327) disposed in the second electric field area;
a fifth membrane (328) disposed between the third electrode zone (331) and the fourth membrane (327) so as to define a third interstitial volume (336) therebetween, wherein the third interstitial volume (336) is separated from the third and fourth electrode zones (331, 332) by the fourth and fifth membranes (327, 328), and wherein at least one of the fourth or fifth membranes (327, 328) is a barrier capable of controlling substantial bulk movement of liquid under the influence of an electric field;
means adapted to communicate liquid to the third electrode zone (331) and the fourth electrode zone (332); and
means adapted to communicate a sample constituent from the first or second interstitial volume (316, 326) to the third interstitial volume (336).

**17.** The process of any one of claims 14 to 16, wherein the apparatus further comprises:

means adapted for removing heat generated in the apparatus.

**18.** The process of any one of claims 14 to 17, wherein the inducible electro-endo-osmotic membrane is selected from the group consisting of cellulose tri-acetate, with a molecular mass cut-off from 5 to 20 kDa polyvinyl alcohol, and poly(vinyl alcohol) cross-linked with glutaraldehyde.

**19.** The process of claim 18, wherein the inducible electro-endo-osmotic membrane has a defined pore size.

**20.** The process of claim 19, wherein the inducible electro-endo-osmotic membrane is made from polyvinyl alcohol or polyvinyl alcohol cross-linked with glutaraldehyde and has a defined pore size from about 5 to about 1000 kDa.

**21.** The process of any one of claims 14 to 20, wherein the inducible electro-endo-osmotic membrane is adjacent to one of the electrode zones.

**22.** The process of any one of claims 14 to 21, wherein one or more membranes are electrophoresis separation membranes having defined pore sizes.

**23.** The process of claim 22, wherein the electrophoresis separation membranes are made from polyacrylamide.

**24.** The process of claim 23, wherein the electrophoresis separation membranes have defined pore sizes from about 1 to about 2000 kDa.

**25.** The process of any one of claims 14 to 24, further comprising the steps of:

(a) adding buffer or solvent to the first and second electrode zones (111, 112);
(b) adding buffer or solvent to the third and fourth electrode zones, if present;
(c) adding the sample to the first interstitial volume (116);
(d) applying an electric potential between the electrodes (113, 114) causing liquid in the sample to move through a membrane into an adjacent electrode zone (111, 112); and
(e) optionally obtaining the concentrated sample;

wherein the barrier membrane allows bulk movement of liquid out of the sample, and wherein the sample is concentrated.

**26.** The process of claim 15, or any one of claims 16 to 24 when dependent on claim 15, further comprising the steps of:

(a) adding buffer or solvent to the first and second electrode zones (211, 212);
(b) adding buffer or solvent to the third and fourth electrode zones, if present;
(c) adding the sample to the first interstitial volume (216);
(d) adding buffer or liquid to the second interstitial volume (226);
(e) applying an electric potential between the electrodes (213, 214) causing at least one compound from the sample to move through a membrane into an adjacent electrode zone or interstitial volume; and
(f) optionally collecting the compound moved from the sample;

wherein the barrier membrane controls substantial bulk movement of liquid into or out of one or more of the interstitial volumes, and wherein at least one component from a sample is moved while controlling the bulk movement of liquid.

**27.** The process of claim 16, or any one of claims 17 to 24 when dependent on claim 16, further comprising the steps of:

(a) adding buffer or electrolyte to the first, second, third and fourth electrode zones (311, 312, 331, 332);
(b) adding the sample to the first interstitial volume (316);
(c) adding buffer or liquid to the second interstitial volume (326);
(d) applying a first electric potential between the first and second electrodes (313, 314) causing at least one compound from the sample to move through a membrane into the second interstitial volume (326);
(e) passing sample containing the compound from either the first or second interstitial volume (316, 326) to the third interstitial volume (336);

(f) applying a second electric potential between the third and fourth electrodes (323, 324) causing liquid in the third interstitial volume (336) to move through a membrane into an adjacent electrode zone (331, 332) thereby concentrating the compound in the sample; and

(g) optionally collecting the concentrated sample;

wherein the barrier membrane controls substantial bulk movement of liquid into the third interstitial volume (336), and wherein at least one component from a sample is moved while controlling the bulk movement of liquid.

**Patentansprüche**

1. Elektrophoresevorrichtung (100) umfassend:

eine erste Elektrode (113) in einer ersten Elektrodenzone (111);

eine zweite Elektrode (114) in einer zweiten Elektrodenzone (112), wobei die erste Elektrode (113) relativ zu der zweiten Elektrode (114) so angeordnet ist, dass sie angepasst ist, um dazwischen ein elektrisches Feld in einem elektrischen Feldbereich nach Anlegen eines elektrischen Potenzials zwischen der ersten und der zweiten Elektrode (113, 114) zu erzeugen;

eine erste Membran (117), die in dem elektrischen Feldbereich angeordnet ist,

eine zweite Membran (118), die zwischen der ersten Elektrodenzone (111) und der ersten Membran (117) so angeordnet ist, um dazwischen ein erstes Zwischenvolumen (116) zu definieren, wobei das erste Zwischenvolumen (116) von der ersten und zweiten Elektrodenzone (111, 112) durch die erste und zweite Membran (117, 118) getrennt ist;

Mittel (119, 120), die angepasst sind, um Flüssigkeit zu der ersten Elektrodenzone (111) und der zweiten Elektrodenzone (112) zu übertragen; und

Mittel (121), die angepasst sind, um einen Probenbestandteil zu dem ersten Zwischenvolumen (116) zu übertragen;

**dadurch gekennzeichnet, dass** wenigstens eine Membran eine Barriere in der Form einer induzierbaren elektro-endo-osmotischen Membran ist, die in der Lage ist, eine wesentliche Flüssigkeitsvolumenbewegung unter dem Einfluss eines elektrischen Feldes zu steuern, wobei die wenigstens eine induzierbare elektro-endo-osmotische Membran entweder

(a) aus Zellulosetriacetat mit einem Molekülmassenausschluss von 5 bis 20 kDa hergestellt ist, oder

(b) aus Polyvinylalkohol hergestellt ist.

2. Vorrichtung (200) nach Anspruch 1, weiter umfassend:

eine dritte Membran (222), die zwischen einer zweiten Elektrodenzone (212) und der ersten Membran (217) angeordnet ist, um dazwischen ein zweites Zwischenvolumen (226) zu definieren, wobei das erste Zwischenvolumen (216) von der ersten Elektrodenzone (211) durch die zweite Membran (218) getrennt ist, und das zweite Zwischenvolumen (226) von der zweiten Elektrodenzone (212) durch die dritte Membran (222) getrennt ist.

3. Vorrichtung (300) nach Anspruch 2, weiter umfassend:

eine dritte Elektrode (323) in einer dritten Elektrodenzone (331);

eine vierte Elektrode (324) in einer vierten Elektrodenzone (332), wobei die dritte Elektrode (323) relativ zu der vierten Elektrode (324) so angeordnet ist, dass sie angepasst ist, um dazwischen ein elektrisches Feld in einem zweiten elektrischen Feldbereich nach Anlegen eines zweiten elektrischen Potenzials zwischen der dritten und der vierten Elektrode (323, 324) zu erzeugen;

eine vierte Membran (327), die in dem zweiten elektrischen Feldbereich angeordnet ist;

eine fünfte Membran (328), die zwischen der dritten Elektrodenzone (331) und der vierten Membran (327) angeordnet ist, um dazwischen ein drittes Zwischenvolumen (336) zu definieren, wobei das dritte Zwischenvolumen (336) von der dritten und vierten Elektrodenzone (331, 332) durch die vierte und fünfte Membran (327, 328) getrennt ist, und wobei wenigstens entweder die vierte oder die fünfte Membran (327, 328) eine Barriere ist, die in der Lage ist, eine wesentliche Flüssigkeitsvolumenbewegung unter dem Einfluss eines elektrischen Feldes zu steuern;

Mittel, die angepasst sind, um Flüssigkeiten zu der dritten Elektrodenzone (331) und der vierten Elektrodenzone (332) zu übertragen; und

Mittel, die angepasst sind, um einen Probenbestandteil aus dem ersten oder zweiten Zwischenvolumen (316, 326) zu einem dritten Zwischenvolumen (336) zu übertragen.

4. Vorrichtung nach einem der Ansprüche 1 bis 3, weiter umfassend:

Mittel, die angepasst sind zum Entfernen von in der Vorrichtung erzeugten Wärme.

5. Vorrichtung nach einem der Ansprüche 1 bis 4, wobei die induzierbare elektro-endo-osmotische Membran eine definierte Porengröße aufweist.

6. Vorrichtung nach Anspruch 5, wobei die induzierbare elektro-endo-osmotische Membran aus Polyvinylalkohol hergestellt ist und eine definierte Porengröße von etwa 5 bis etwa 1000 kDa aufweist.

7. Vorrichtung nach einem der Ansprüche 1 bis 6, wobei die induzierbare elektro-endo-osmotische Membran benachbart zu einer der Elektrodenzonen ist.

8. Vorrichtung nach einem der Ansprüche 1 bis 7, wobei eine oder mehrere Membranen Elektrophorese-Trennmembranen mit definierten Porengrößen ist/sind.

9. Vorrichtung nach Anspruch 8, wobei die Elektrophorese-Trennmembranen aus Polyacrylamid hergestellt sind.

10. Vorrichtung nach Anspruch 9, wobei die Elektrophorese-Trennmembranen definierte Porengrößen von etwa 1 bis etwa 2000 kDa aufweisen.

11. Verfahren zum Konzentrieren einer Probe, wobei das Verfahren umfasst:

(a) Bereitstellen einer Elektrophoresevorrichtung (100) gemäß einem der Ansprüche 1 bis 10;
(b) Hinzugeben von Puffer oder Lösungsmittel zu der ersten und zweiten Elektrodenzone (111, 112);
(c) Hinzugeben von Puffer oder Lösungsmittel zu der dritten und vierten Elektrodenzone, sofern vorhanden;
(d) Hinzugeben der Probe zu dem ersten Zwischenvolumen (116);
(e) Anlegen eines elektrischen Potenzials zwischen den Elektroden (113, 114), was bedingt, dass sich Flüssigkeit in der Probe durch eine Membran in eine benachbarte Elektrodenzone (111, 112) bewegt; und
(f) optional Erhalten der konzentrierten Probe;

wobei die Barrierenmembran eine Flüssigkeitsvolumenbewegung aus der Probe heraus erlaubt.

12. Verfahren zum Bewegen wenigstens eines Bestandteils aus einer Probe heraus, während eine Flüssigkeitsvolumenbewegung gesteuert wird, wobei das Verfahren umfasst:

(a) Bereitstellen einer Elektrophoresevorrichtung (200) gemäß Anspruch 2 oder einem der Ansprüche 3 bis 10, sofern sie von Anspruch 2 abhängig sind;
(b) Hinzugeben von Puffer oder Lösungsmittel zu der ersten und zweiten Elektrodenzone (211, 212);
(c) Hinzugeben von Puffer oder Lösungsmittel zu der dritten und vierten Elektrodenzone, sofern vorhanden;
(d) Hinzugeben der Probe zu dem ersten Zwischenvolumen (216);
(e) Hinzugeben von Puffer oder Flüssigkeit zu dem zweiten Zwischenvolumen (226);
(f) Anlegen eines elektrischen Potenzials zwischen den Elektroden (213, 214), was bedingt, dass sich wenigstens eine Verbindung aus der Probe durch eine Membran in eine benachbarte Elektrodenzone oder ein Zwischenvolumen bewegt; und
(g) optional Sammeln der aus der Probe heraus bewegten Verbindung;

wobei die Barrieremembran eine wesentliche Flüssigkeitsvolumenbewegung in eines oder mehrere der Zwischenvolumina hinein oder aus einem oder mehreren der Zwischenvolumina heraus steuert.

13. Verfahren zum Bewegen wenigstens eines Bestandteiles aus einer Probe heraus, während eine Flüssigkeitsvolumenbewegung gesteuert wird, wobei das Verfahren umfasst:

(a) Bereitstellen einer Elektrophoresevorrichtung (300) gemäß Anspruch 3 oder einem der Ansprüche 4 bis 10, sofern sie von Anspruch 3 abhängig sind;

(b) Hinzugeben von Puffer oder Elektrolyt zu der ersten, zweiten, dritten und vierten Elektrodenzone (311, 312, 331, 332);

(c) Hinzugeben der Probe zu dem ersten Zwischenvolumen (316);

(d) Hinzugeben von Puffer oder Flüssigkeit zu dem zweiten Zwischenvolumen (326);

(e) Anlegen eines ersten elektrischen Potenzials zwischen der ersten und zweiten Elektrode (313, 314), was bedingt, dass sich wenigstens eine Verbindung aus der Probe heraus durch eine Membran in das zweite Zwischenvolumen (326) bewegt;

(f) Überführen einer Probe, die die Verbindung enthält, aus entweder dem ersten oder dem zweiten Zwischenvolumen (316, 326) in das dritte Zwischenvolumen (336);

(g) Anlegen eines zweiten elektrischen Potenzials zwischen der dritten und vierten Elektrode (323, 324), was bedingt, dass sich Flüssigkeit in dem dritten Zwischenvolumen (336) durch eine Membran in eine benachbarte Elektrodenzone (331, 332) bewegt, wodurch die Verbindung in der Probe aufkonzentriert wird; und

(h) optional Sammeln der konzentrierten Probe;

wobei die Barrieremembran eine wesentliche Flüssigkeitsvolumenbewegung in das dritte Zwischenvolumen (336) steuert.

**14.** Elektrophoreseverfahren umfassend die Schritte:

(a) Bereitstellen einer Vorrichtung (100) umfassend:

eine erste Elektrode (113) in einer ersten Elektrodenzone (111);

eine zweite Elektrode (114) in einer zweiten Elektrodenzone (112), wobei die erste Elektrode (113) relativ zu der zweiten Elektrode (114) so angeordnet ist, dass sie angepasst ist, um dazwischen ein elektrisches Feld in einem elektrischen Feldbereich nach Anlegen eines elektrischen Potenzials zwischen der ersten und zweiten Elektrode (113, 114) zu erzeugen;

eine ersten Membran (117), die in dem elektrischen Feldbereich angeordnet ist;

eine zweite Membran (118), die zwischen der ersten Elektrodenzone (111) und der ersten Membran (117) angeordnet ist, um dazwischen ein erstes Zwischenvolumen (116) zu definieren, wobei das ersten Zwischenvolumen (116) von der ersten und zweiten Elektrodenzone (111, 112) durch die erste und zweite Membran (117, 118) getrennt ist, und wobei wenigstens eine Membran eine Barriere in der Form einer induzierbaren elektro-endo-osmotischen Membran ist, die in der Lage ist, eine wesentliche Flüssigkeitsvolumenbewegung unter dem Einfluss eines elektrischen Feldes zu steuern;

Mittel (119, 120), die angepasst sind, um Flüssigkeit zu der ersten Elektrodenzone (111) und der zweiten Elektrodenzone (112) zu übertragen; und

Mittel (121), die angepasst sind, um einen Probenbestandteil zu dem ersten Zwischenvolumen (116) zu übertragen; und

(b) Steuern einer wesentlichen Flüssigkeitsvolumenbewegung unter dem Einfluss eines elektrischen Feldes mit der wenigstens einen induzierbaren elektro-endo-osmotischen Membran.

**15.** Verfahren nach Anspruch 14, wobei die Vorrichtung (200) weiter umfasst:

eine dritte Membran (222), die zwischen einer zweiten Elektrodenzone (212) und der ersten Membran (217) angeordnet ist, um dazwischen ein zweites Zwischenvolumen (226) zu definieren, wobei das erste Zwischenvolumen (216) von der ersten Elektrodenzone (211) durch die zweite Membran (218) getrennt ist, und das zweite Zwischenvolumen (226) von der zweiten Elektrodenzone (212) durch die dritte Membran (222) getrennt ist.

**16.** Verfahren nach Anspruch 15, wobei die Vorrichtung (300) weiter umfasst:

eine dritte Elektrode (323) in einer dritten Elektrodenzone (331);

eine vierte Elektrode (324) in einer vierten Elektrodenzone (332), wobei die dritte Elektrode (323) relativ zu der vierten Elektrode (324) so angeordnet ist, dass sie angepasst ist, um dazwischen ein elektrisches Feld in einem zweiten elektrischen Feldbereich nach Anlegen eines zweiten elektrischen Potenzials zwischen der dritten und vierten Elektrode (323, 324) zu erzeugen;

eine vierte Membran (327), die in dem zweiten elektrischen Feldbereich angeordnet ist;

eine fünfte Membran (328), die zwischen der dritten Elektrodenzone (331) und der vierten Membran (327) angeordnet ist, um dazwischen ein drittes Zwischenvolumen (336) zu definieren, wobei das dritte Zwischenvolumen (336) von der dritten und vierten Elektrodenzone (331, 332) durch die vierte und fünfte Membran (327, 328) getrennt ist, und wobei wenigstens entweder die vierte oder fünfte Membran (327, 328) eine Barriere ist, die in der Lage ist, eine wesentliche Flüssigkeitsvolumenbewegung unter dem Einfluss eines elektrischen Feldes zu steuern;

Mittel, die angepasst sind, um Flüssigkeit zu der dritten Elektrodenzone (331) und zur vierten Elektrodenzone (332) zu übertragen; und

Mittel, die angepasst sind, um einen Probenbestandteil aus dem ersten oder zweiten Zwischenvolumen (316, 326) zu dem dritten Zwischenvolumen (336) zu übertragen.

**17.** Verfahren nach einem der Ansprüche 14 bis 16, wobei die Vorrichtung weiter umfasst:

Mittel, die angepasst sind zum Abführen von Wärme, die in der Vorrichtung erzeugt wird.

**18.** Verfahren nach einem der Ansprüche 14 bis 17, wobei die induzierbare elektro-endo-osmotische Membran ausgewählt ist aus der Gruppe bestehend aus Zellulosetriacetat mit einem Molekülmassenausschluss von 5 bis 20 kDa, Polyvinylalkohol und mit Glutaraldehyd quervernetztem Poly(vinylalkohol).

**19.** Verfahren nach Anspruch 18, wobei die induzierbare elektro-endo-osmotische Membran eine definierte Porengröße aufweist.

**20.** Verfahren nach Anspruch 19, wobei die induzierbare elektro-endo-osmotische Membran hergestellt ist aus Polyvinylalkohol oder mit Glutaraldehyd quervernetztem Polyvinylalkohol und eine definierte Porengröße von etwa 5 bis etwa 1000 kDa aufweist.

**21.** Verfahren nach einem der Ansprüche 14 bis 20, wobei die induzierbare elektro-endo-osmotische Membran benachbart zu einer der Elektrodenzonen ist.

**22.** Verfahren nach einem der Ansprüche 14 bis 21, wobei eine oder mehrere Membranen Elektrophorese-Trennmembranen mit definierten Porengrößen sind.

**23.** Verfahren nach Anspruch 22, wobei die Elektrophorese-Trennmembranen aus Polyacrylamid hergestellt sind.

**24.** Verfahren nach Anspruch 23, wobei die Elektrophorese-Trennmembranen definierte Porengrößen von etwa 1 bis etwa 2000 kDa aufweisen.

**25.** Verfahren nach einem der Ansprüche 14 bis 21, weiter umfassend die Schritte:

(a) Hinzugeben von Puffer oder Lösungsmittel zu der ersten und zweiten Elektrodenzone (111, 112);
(b) Hinzugeben von Puffer oder Lösungsmittel zu der dritten und vierten Elektrodenzone, sofern vorhanden;
(c) Hinzugeben der Probe zu dem ersten Zwischenvolumen (116);
(d) Anlegen eines elektrischen Potenzials zwischen den Elektroden (113, 114), was bedingt, dass sich Flüssigkeit in der Probe durch eine Membran in eine benachbarte Elektrodenzone (111, 112) bewegt; und
(e) optional Erhalten der konzentrierten Probe;

wobei die Barrierenmembran eine Flüssigkeitsvolumenbewegung aus der Probe erlaubt und die Probe konzentriert wird.

**26.** Verfahren nach Anspruch 15 oder einem der Ansprüche 16 bis 24, sofern sie von Anspruch 15 abhängig sind, weiter umfassend die Schritte:

(a) Hinzugeben von Puffer oder Lösungsmittel zu der ersten und zweiten Elektrodenzone (211, 212);
(b) Hinzugeben von Puffer oder Lösungsmittel zu der dritten und vierten Elektrodenzone, sofern vorhanden;
(c) Hinzugeben der Probe zu dem ersten Zwischenvolumen (216);
(d) Hinzugeben von Puffer oder Flüssigkeit zu dem zweiten Zwischenvolumen (226);
(e) Anlegen eines elektrischen Potenzials zwischen den Elektroden (213, 214), was bedingt, dass sich wenigstens eine Verbindung aus der Probe durch eine Membran in eine benachbarte Elektrodenzone oder Zwischenvolumen bewegt; und

(f) optional Sammeln der Verbindung, die sich aus der Probe bewegte;

wobei die Barrierenmembran eine wesentliche Flüssigkeitsvolumenbewegung in eines oder mehrere der Zwischenvolumina hinein oder aus einem oder mehreren der Zwischenvolumina heraus steuert, und wobei sich wenigstens ein Bestandteil aus einer Probe bewegt, während eine Flüssigkeitsvolumenbewegung gesteuert wird.

27. Verfahren nach Anspruch 16, oder einem der Ansprüche 17 bis 24, sofern sie von Anspruch 16 abhängig sind, weiter umfassend die Schritte:

(a) Hinzugeben von Puffer oder Elektrolyt zu der ersten, zweiten, dritten und vierten Elektrodenzone (311, 312, 331, 332);
(b) Hinzugeben der Probe zu dem ersten Zwischenvolumen (316);
(c) Hinzugeben von Puffer oder Flüssigkeit zu dem zweiten Zwischenvolumen (326);
(d) Anlegen eines ersten elektrischen Potenzials zwischen der ersten und zweiten Elektrode (313, 314), was bedingt, das sich wenigstens eine Verbindung aus der Probe durch eine Membran in das zweite Zwischenvolumen (326) bewegt;
(e) Durchführen einer Probe, die die Verbindung enthält, aus entweder dem ersten oder zweiten Zwischenvolumen (316, 326) in das dritte Zwischenvolumen (336);
(f) Anlegen eines zweiten elektrischen Potenzials zwischen der dritten und vierten Elektrode (323, 324), was bedingt, dass sich Flüssigkeit in dem dritten Zwischenvolumen (336) durch eine Membran in eine benachbarte Elektrodenzone (331, 332) bewegt, wodurch die Verbindung in der Probe aufkonzentriert wird; und
(g) optional Sammeln der konzentrierten Probe;

wobei die Barrierenmembran eine wesentliche Flüssigkeitsvolumenbewegung in das dritte Zwischenvolumen (336) steuert; und wobei wenigstens ein Bestandteil einer Probe bewegt wird, während eine Flüssigkeitsvolumenbewegung gesteuert wird.

## Revendications

1. Appareil d'électrophorèse (100) comprenant :

une première électrode (113) dans une première zone d'électrode (111) ;
une seconde électrode (114) dans une seconde zone d'électrode (112), la première électrode (113) étant disposée par rapport à la seconde électrode (114) de façon à être adaptée afin de générer un champ électrique dans une zone de champ électrique située entre celles-ci lors de l'application d'un potentiel électrique entre la première et la seconde électrodes (113, 114) ;
une première membrane (117) disposée dans la zone de champ électrique ;
une seconde membrane (118) disposée entre la première zone d'électrode (111) et la première membrane (117) de façon à définir un premier volume interstitiel (116) entre celles-ci, dans laquelle le premier volume interstitiel (116) est séparé de la première et de la seconde zones d'électrode (111, 112) par la première et la seconde membranes (117, 118) ;
un moyen (119, 120) adapté pour communiquer un liquide à la première zone d'électrode (111) et à la seconde zone d'électrode (112) ; et
un moyen (121) adapté pour communiquer un constituant d'échantillon au premier volume interstitiel (116) ;

**caractérisé en ce qu'**au moins une membrane est une barrière sous la forme d'une membrane électro-endo-osmotique inductible capable de contrôler un mouvement de vrac substantiel du liquide sous l'influence d'un champ électrique, dans laquelle la membrane électro-endo-osmotique inductible au moins est soit

(a) constituée de triacétate de cellulose ayant une masse moléculaire de 5 à 20 kDa, soit
(b) constituée d'alcool polyvinylique.

2. Appareil (200) selon la revendication 1, comprenant en outre :

une troisième membrane (222) disposée entre une seconde zone d'électrode (212) et la première membrane (217) de façon à définir un second volume interstitiel (226) entre celles-ci, dans laquelle le premier volume interstitiel (216) est séparé de la première zone d'électrode (211) par la seconde membrane (218) et le second volume interstitiel (226) est séparé de la seconde zone d'électrode (212) par la troisième membrane (222).

**3.** Appareil (300) selon la revendication 2, comprenant en outre :

une troisième électrode (323) dans une troisième zone d'électrode (331) ;
une quatrième électrode (324) dans une quatrième zone d'électrode (332), la troisième électrode (323) étant disposée par rapport à la quatrième électrode (324) de façon à être adaptée afin de générer un champ électrique dans une seconde zone de champ électrique entre celles-ci lors de l'application d'un second potentiel électrique entre la troisième et la quatrième électrodes (323, 324) ;
une quatrième membrane (327) disposée dans la seconde zone de champ électrique ;
une cinquième membrane (328) disposée entre la troisième zone d'électrode (331) et la quatrième membrane (327) de façon à définir un troisième volume interstitiel (336) entre celles-ci, dans laquelle le troisième volume interstitiel (336) est séparé de la troisième et de la quatrième zones d'électrode (331, 332) par la quatrième et la cinquième membranes (327, 328), et dans laquelle au moins l'une de la quatrième ou de la cinquième membrane (327, 328) est une barrière capable de contrôler un mouvement de vrac substantiel de liquide sous l'influence d'un champ électrique ;
un moyen adapté pour communiquer un liquide à la troisième zone d'électrode (331) et à la quatrième zone d'électrode (332) ; et
un moyen adapté pour communiquer un constituant d'échantillon entre le premier ou le second volume interstitiel (316, 326) et le troisième volume interstitiel (336).

**4.** Appareil selon l'une quelconque des revendications 1 à 3, comprenant en outre :

un moyen adapté pour supprimer la chaleur générée dans l'appareil.

**5.** Appareil selon l'une quelconque des revendications 1 à 4, dans lequel la membrane électro-endo-osmotique inductible possède une taille de pores définie.

**6.** Appareil selon la revendication 5, dans lequel la membrane électro-endo-osmotique inductible est constituée d'alcool polyvinylique et possède une taille de pores définie d'environ 5 à environ 1000 kDa.

**7.** Appareil selon l'une quelconque des revendications 1 à 6, dans lequel la membrane électro-endo-osmotique inductible est adjacente à l'une des zones d'électrode.

**8.** Appareil selon l'une quelconque des revendications 1 à 7, dans lequel une ou plusieurs membrane(s) est/sont une/des membrane(s) de séparation par électrophorèse ayant des tailles de pores définies.

**9.** Appareil selon la revendication 8, dans lequel les membranes de séparation par électrophorèse sont constituées de polyacrylamide.

**10.** Appareil selon la revendication 9, dans lequel les membranes de séparation par électrophorèse possèdent des tailles de pores définies d'environ 1 à environ 2000 kDa.

**11.** Procédé de concentration d'un échantillon, le procédé comprenant :

(a) le fait de prévoir un appareil d'électrophorèse (100) selon l'une quelconque des revendications 1 à 10 ;
(b) l'ajout d'un tampon ou d'un solvant à la première et la seconde zones d'électrode (111 ; 112) ;
(c) l'ajout d'un tampon ou d'un solvant à la troisième et la quatrième zones d'électrode, le cas échéant ;
(d) l'ajout de l'échantillon au premier volume interstitiel (116) ;
(e) l'application d'un potentiel électrique entre les électrodes (113, 114) provoquant le fait que le liquide situé dans l'échantillon se déplace à travers une membrane dans une zone d'électrode adjacente (111, 112) ; et
(f) l'obtention optionnelle de l'échantillon concentré ;

dans lequel la membrane barrière permet un mouvement de vrac du liquide en-dehors de l'échantillon.

**12.** Procédé de déplacement d'au moins un composant d'un échantillon tout en contrôlant le mouvement de vrac d'un liquide, le procédé comprenant :

(a) le fait de prévoir un appareil d'électrophorèse (200) selon la revendication 2 ou l'une quelconque des revendications 3 à 10 lorsqu'elles dépendent de la revendication 2 ;
(b) l'ajout d'un tampon ou d'un solvant à la première et la seconde zones d'électrode (211, 212) ;
(c) l'ajout d'un tampon ou d'un solvant à la troisième et la quatrième zones d'électrode, le cas échéant ;
(d) l'ajout de l'échantillon au premier volume interstitiel (216) ;
(e) l'ajout d'un tampon ou d'un liquide au second volume interstitiel (226) ;
(f) l'application d'un potentiel électrique entre les électrodes (213, 214) provoquant le fait qu'au moins un composé de l'échantillon se déplace à travers une membrane dans une zone d'électrode adjacente ou un volume interstitiel adjacent ; et
(g) la collecte optionnelle du composé déplacé de l'échantillon ;

dans lequel la membrane barrière contrôle le mouvement de vrac substantiel du liquide dans ou en-dehors d'un ou plusieurs des volumes interstitiels.

13. Procédé de déplacement d'au moins un composant d'un échantillon tout en contrôlant le mouvement de vrac du liquide, le procédé comprenant :

(a) le fait de prévoir un appareil d'électrophorèse (300) selon la revendication 3 ou l'une quelconque des revendications 4 à 10 lorsqu'elles dépendent de la revendication 3 ;
(b) l'ajout d'un tampon ou d'un électrolyte à la première, la seconde, la troisième et la quatrième zones d'électrode (311, 312, 331, 332) ;
(c) l'ajout de l'échantillon au premier volume interstitiel (316) ;
(d) l'ajout d'un tampon ou d'un liquide au second volume interstitiel (326) ;
(e) l'application d'un premier potentiel électrique entre la première et la seconde électrodes (313, 314) provoquant le fait qu'au moins un composé de l'échantillon se déplace à travers une membrane dans le second volume interstitiel (326) ;
(f) le passage de l'échantillon contenant le composé entre le premier ou le second volume interstitiel (316, 326) et le troisième volume interstitiel (336) ;
(g) l'application d'un second potentiel électrique entre la troisième et la quatrième électrodes (323, 324) provoquant le fait que le liquide situé dans le troisième volume interstitiel (336) se déplace à travers une membrane dans une zone d'électrode adjacente (331, 332), concentrant ainsi le composé dans l'échantillon ; et
(h) la collecte optionnelle de l'échantillon concentré;

dans lequel la membrane barrière contrôle le mouvement de vrac substantiel du liquide dans le troisième volume interstitiel (336).

14. Processus d'électrophorèse comprenant les étapes consistant à :

(a) prévoir un appareil (100) comprenant :

une première électrode (113) dans une première zone d'électrode (111) ;
une seconde électrode (114) dans une seconde zone d'électrode (112), la première électrode (113) étant disposée par rapport à la seconde électrode (114) de façon à être adaptée afin de générer un champ électrique dans une zone de champ électrique située entre celles-ci lors de l'application d'un potentiel électrique entre la première et la seconde électrodes (113, 114) ;
une première membrane (117) disposée dans la zone de champ électrique ;
une seconde membrane (118) disposée entre la première zone d'électrode (111) et la première membrane (117) de façon à définir un premier volume interstitiel (116) entre celles-ci, dans laquelle le premier volume interstitiel (116) est séparé de la première et de la seconde zones d'électrode (111, 112) par la première et la seconde membranes (117, 118), et dans laquelle au moins une membrane est une barrière sous la forme d'une membrane électro-endo-osmotique inductible capable de contrôler un mouvement de vrac substantiel du liquide sous l'influence d'un champ électrique ;
un moyen (119, 120) adapté pour communiquer un liquide à la première zone d'électrode (111) et à la seconde zone d'électrode (112) ; et
un moyen (121) adapté pour communiquer un constituant d'échantillon au premier volume interstitiel (116) ; et

(b) contrôler le mouvement de vrac substantiel du liquide sous l'influence d'un champ électrique avec la membrane électro-endo-osmotique inductible au moins.

**15.** Processus selon la revendication 14, dans lequel l'appareil (200) comprend en outre :

une troisième membrane (222) disposée entre une seconde zone d'électrode (212) et la première membrane (217) de façon à définir un second volume interstitiel (226) entre celles-ci, dans laquelle le premier volume interstitiel (216) est séparé de la première zone d'électrode (211) par la seconde membrane (218) et le second volume interstitiel (226) est séparé de la seconde zone d'électrode (212) par la troisième membrane (222).

**16.** Processus selon la revendication 15, dans lequel l'appareil (300) comprend en outre :

une troisième électrode (323) dans une troisième zone d'électrode (331);
une quatrième électrode (324) dans une quatrième zone d'électrode (332), la troisième électrode (323) étant disposée par rapport à la quatrième électrode (324) de façon à être adaptée afin de générer un champ électrique dans une seconde zone de champ électrique entre celles-ci lors de l'application d'un second potentiel électrique entre la troisième et la quatrième électrodes (323, 324) ;
une quatrième membrane (327) disposée dans la seconde zone de champ électrique ;
une cinquième membrane (328) disposée entre la troisième zone d'électrode (331) et la quatrième membrane (327) de façon à définir un troisième volume interstitiel (336) entre celles-ci, dans laquelle le troisième volume interstitiel (336) est séparé de la troisième et de la quatrième zones d'électrode (331, 332) par la quatrième et la cinquième membranes (327, 328), et dans laquelle au moins l'une de la quatrième ou de la cinquième membrane (327, 328) est une barrière capable de contrôler un mouvement de vrac substantiel de liquide sous l'influence d'un champ électrique ;
un moyen adapté pour communiquer un liquide à la troisième zone d'électrode (331) et à la quatrième zone d'électrode (332) ; et
un moyen adapté pour communiquer un constituant d'échantillon entre le premier ou le second volume interstitiel (316, 326) et le troisième volume interstitiel (336).

**17.** Processus selon l'une quelconque des revendications 14 à 16, dans lequel l'appareil comprend en outre :

un moyen adapté pour supprimer la chaleur générée dans l'appareil.

**18.** Processus selon l'une quelconque des revendications 14 à 17, dans lequel la membrane électro-endo-osmotique inductible est choisie parmi le groupe consistant en triacétate de cellulose ayant une masse moléculaire de 5 à 20 kDa, en alcool polyvinylique, et en alcool poly(vinylique) réticulé avec du glutaraldéhyde.

**19.** Processus selon la revendication 18, dans lequel la membrane électro-endo-osmotique inductible possède une taille de pores définie.

**20.** Processus selon la revendication 19, dans lequel la membrane électro-endo-osmotique inductible est constituée d'alcool polyvinylique ou d'alcool polyvinylique réticulé avec du glutaraldéhyde et possède une taille de pores définie d'environ 5 à environ 1000 kDa.

**21.** Processus selon l'une quelconque des revendications 14 à 20, dans lequel la membrane électro-endo-osmotique inductible est adjacente à l'une des zones d'électrode.

**22.** Processus selon l'une quelconque des revendications 14 à 21, dans lequel une ou plusieurs membrane(s) est/sont une/des membrane(s) de séparation par électrophorèse ayant des tailles de pores définies.

**23.** Processus selon la revendication 22, dans lequel les membranes de séparation par électrophorèse sont constituées de polyacrylamide.

**24.** Processus selon la revendication 23, dans lequel les membranes de séparation par électrophorèse possèdent des tailles de pores définies d'environ 1 à environ 2000 kDa.

**25.** Processus selon l'une quelconque des revendications 14 à 24, comprenant en outre les étapes consistant à :

(a) ajouter un tampon ou un solvant à la première et la seconde zones d'électrode (111, 112) ;

(b) ajouter un tampon ou un solvant à la troisième et la quatrième zones d'électrode, le cas échéant ;
(c) ajouter l'échantillon au premier volume interstitiel (116) ;
(d) appliquer un potentiel électrique entre les électrodes (113, 114) provoquant le fait que le liquide situé dans l'échantillon se déplace à travers une membrane dans une zone d'électrode adjacente (111, 112) ; et
(e) obtenir optionnellement l'échantillon concentré ;

dans lequel la membrane barrière permet un mouvement de vrac du liquide en-dehors de l'échantillon, et dans lequel l'échantillon est concentré.

26. Processus selon la revendication 15, ou l'une quelconque des revendications 16 à 24 lorsqu'elles dépendent de la revendication 15, comprenant en outre les étapes consistant à :

(a) ajouter un tampon ou un solvant à la première et la seconde zones d'électrode (211, 212) ;
(b) ajouter un tampon ou un solvant à la troisième et la quatrième zones d'électrode, le cas échéant ;
(c) ajouter l'échantillon au premier volume interstitiel (216) ;
(d) ajouter un tampon ou un liquide au second volume interstitiel (226) ;
(e) appliquer un potentiel électrique entre les électrodes (213, 214) provoquant le fait qu'au moins un composé de l'échantillon se déplace à travers une membrane dans une zone d'électrode adjacente ou un volume interstitiel adjacent ; et
(f) collecter optionnellement le composé déplacé de l'échantillon ;

dans lequel la membrane barrière contrôle le mouvement de vrac substantiel du liquide dans ou en-dehors d'un ou plusieurs des volumes interstitiels, et dans lequel au moins un composant d'un échantillon est déplacé tout en contrôlant le mouvement de vrac du liquide.

27. Processus selon la revendication 16, ou l'une quelconque des revendications 17 à 24 lorsqu'elles dépendent de la revendication 16, comprenant en outre les étapes consistant à :

(a) ajouter un tampon ou un électrolyte à la première, la seconde, la troisième et la quatrième zones d'électrode (311, 312, 331, 332) ;
(b) ajouter l'échantillon au premier volume interstitiel (316) ;
(c) ajouter un tampon ou un liquide au second volume interstitiel (326) ;
(d) appliquer un premier potentiel électrique entre la première et la seconde électrodes (313, 314) provoquant le fait qu'au moins un composé de l'échantillon se déplace à travers une membrane dans le second volume interstitiel (326) ;
(e) faire passer l'échantillon contenant le composé entre le premier ou le second volume interstitiel (316, 326) et le troisième volume interstitiel (336) ;
(f) appliquer un second potentiel électrique entre la troisième et la quatrième électrodes (323, 324) provoquant le fait que le liquide situé dans le troisième volume interstitiel (336) se déplace à travers une membrane dans une zone d'électrode adjacente (331, 332), concentrant ainsi le composé dans l'échantillon ; et
(g) collecter optionnellement l'échantillon concentré ;

dans lequel la membrane barrière contrôle le mouvement de vrac substantiel du liquide dans le troisième volume interstitiel (336), et dans lequel au moins un composant d'un échantillon est déplacé tout en contrôlant le mouvement de vrac du liquide.

Figure 1A

Figure 1B

**Figure 2A**

**Figure 2B**

Figure 3A

Figure 3B

Figure 4

| 10 kDa CTA | |
|---|---|
| 1 2 3 4 5 | 1- Marker |
| | 2- S1 0 |
| | 3- S2 0 |
| BSA- | 4- S1 45 |
| Ova- | 5- S2 45 |
| | |
| Tryp- | |

| 20 kDa CTA | |
|---|---|
| 1 2 3 4 5 | 1- Marker |
| | 2- S1 0 |
| | 3- S2 0 |
| BSA- | 4- S1 45 |
| Ova- | 5- S2 45 |
| | |
| Tryp- | |

## Rates of Endo-Osmosis With CTA Membranes

Rate (mL/min)

1.60
1.40
1.20
1.00
0.80
0.60
0.40
0.20
0.00

5k CTA    10k CTA    20k CTA

**Experiments**

▦ 2 stream U/S
■ 2 stream D/S
☐ Dialysis

Figure 5

Figure 6

## Comparison Of CTA Orientation and The Endo-Osmotic Rate

Rate (mL/min)

1.20
1.00
0.80
0.60
0.40
0.20
0.00

5k CTA    10k CTA    20k CTA

**Experiment**

▦ S1 Shiny Side Down
■ S1 Shiny Side Up

Figure 7

Rate of Volume Variation - Voltage (CTA)

Figure 8

BSA Recovery

## Figure 9

Rate of Volume Variation - Voltage (PVAl)

## Figure 10

Rate Variation - pH

Buffer Used

acetic acid/GABA          HEPES/imidazole

Figure 11

Machine 2

Single stream

Concentrator

Machine 1

standard 2 stream

configuration

Figure 12

**Comparison Of Endo-Osmosis Rate and % Fibrinogen Recovery**

Figure 13

## Figure 14 Configuration 1

Legend:

→ feed stream

··········▷ Contaminants

→ Excess water

– – –▷ Purified product

⟶ Endo-osmitic
membrane

⟶ normal membrane

## Figure 15

Antibody band
becomes more
concentrated

Relative concentration of
contaminants is decreased
(partial purification)

| 1  | MW Markers |
|----|-----------|
| 2  | Feedstream 0min |
| 3  | Feedstream 30min |
| 4  | Feedstream 60min |
| 5  | Feedstream 90min |
| 6  | Feedstream 120min |
| 7  | Feedstream 150min |
| 8  | Feedstream 180min |
| 9  | Feedstream 180min |
| 10 | IgG standard |

## Figure 16

1   2   3   4   5   6   7   8   9   10

Contaminants transferred to the second stream results in relative purification of the target protein retained in the feedstream

| 1 | MA Marker |
| 2 | Contaminant stream 0min |
| 3 | Contaminant stream 30min |
| 4 | Contaminant stream 60min |
| 5 | Contaminant stream 90min |
| 6 | Contaminant stream 120min |
| 7 | Contaminant stream 150min |
| 8 | Contaminant stream 180min |
| 9 | Tissue culture medium without IgG |
| 10 | Tissue culture medium with IgG (equivalent to Feedstream 0min) |

Figure 17  Configuration 2

Legend:

| | | | |
|---|---|---|---|
| ───────► | feed stream | •••••••••► | Contaminants |
| ───────► | Excess water | ─ ─ ─► | Purified product |
| ◁▨▨▨▨▨▨▨▨▨───── | Endo-osmitic membrane | ◁▨▨▨▨▨▨▨───── | normal membrane |

Figure 18

Target protein is transferred to product
stream and depleted in feedstream. Transfer
rate was maintained by EEO based
concentration of the feedstream

Contaminant concentrations
were reduced in the feedstream
by transfer to the buffer stream
while EEO was used to reduce
feedstream volume

| 1 | Marker |
| 2 | Feedstream 0min |
| 3 | Feedstream 30min |
| 4 | Feedstream 60min |
| 5 | Feedstream 90min |
| 6 | Feedstream 120min |
| 7 | Feedstream 150min |
| 8 | Feedstream 180min |

Figure 19

Purified ands concentrated
antibody in product stream.

| | |
|---|---|
| 1 | Product stream 0min |
| 2 | Product stream 30min |
| 3 | Product stream 60min |
| 4 | Product stream 90min |
| 5 | Product stream 120min |
| 6 | Product stream 150min |
| 7 | Product stream 180min |

Figure 20  Configuration 3

Legend:

| | | | | |
|---|---|---|---|---|
| ——————▶ | feed stream | •••••••••▶ | Contaminants |
| ——————▶ | Excess water | — — —▶ | Purified product |
| ⟋⟍⟍⟍⟍⟍⟍⟍⟍⟍ | Endo-osmitic membrane | ⟋⟍⟍⟍⟍⟍⟍⟍ | normal membrane |

Figure 21 Configuration 4

Legend:

| | | | |
|---|---|---|---|
| ——→ | feed stream | ••••••••••> | Contaminants |
| ——→ | Excess water | – – –> | Purified product |
| ⬯⬯⬯⬯ | Endo-osmitic membrane | ⬯⬯⬯⬯ | normal membrane |

Figure 22 Configuration 5

Legend:

→ feed stream

→ Excess water

⌒⌒⌒⌒⌒ Endo-osmitic membrane

•••••••••> Contaminants

– – –> Purified product

⌒⌒⌒⌒⌒ normal membrane

## Figure 23 Configuration 6

Legend:

→ feed stream                    •••••••••> Contaminants

→ Excess water                   – – –> Purified product

〰〰 Endo-osmitic          〰〰 normal membrane
membrane

Figure 24 Configuration 7

Legend:

| | | | |
|---|---|---|---|
| ———→ | feed stream | •••••••••▷ | Contaminants |
| ———→ | Excess water | — — —▷ | Purified product |
| ⊂⊃⊃⊃⊃⊃⊃⊃⊃⊃⊃◁ | Endo-osmitic membrane | ▰▰▰▰▰▰▰▰▰◁ | normal membrane |